# EUROPEAN PATENT SPECIFICATION

(11) **EP 3 322 757 B1**
(45) Date of publication and mention of the grant of the patent: **26.08.2020**
(21) Application number: 16741849.0
(22) Date of filing: 08.07.2016
(51) Int. Cl.: C09D 5/00, C09D 163/00, C07C 43/225

(54) **TRACER SUBSTANCES FOR SVC ANALYSIS**
INDIKATORSUBSTANZEN ZUR FÜR SVC-ANALYSEN
SUBSTANCES DE MARQUAGE POUR L'ANALYSE DE SVC

(30) Priority: 15.07.2015 EP 15176766
(43) Date of publication of application: 23.05.2018
(73) Proprietor: TWO Teknik ApS, 4660 Store Heddinge (DK)
(72) Inventor: WARNER, Tim, 4660 Store Heddinge (DK); SCHAUMBURG, Kjeld, 2730 Herlev (DK)
(74) Representative: AWA Denmark A/S
(86) International application number: PCT/DK2016/050244
(87) International publication number: WO 2017/008815

(56) References cited:
- WO-A1-2013/079521
- GB-A- 2 462 668
- US-A1- 2011 027 591
- KUBWABO C ET AL: "Quantitative determination of free and total bisphenol A in human urine using labeled BPA glucuronide and isotope dilution mass spectrometry", ANALYTICAL AND BIOANALYTICAL CHEMISTRY, SPRINGER, DE, vol. 406, no. 18, 1 July 2014 (2014-07-01) , pages 4381-4392, XP002752584, ISSN: 1618-2642, DOI: 10.1007/S00216-014-7829-1 [retrieved on 2014-05-11]
- KAWAGUCHI M ET AL: "Molecularly imprinted solid phase extraction using stable isotope labeled compounds as template and liquid chromatography-mass spectrometry for trace analysis of bisphenol A in water sample", ANALYTICA CHIMICA ACTA, ELSEVIER, AMSTERDAM, NL, vol. 539, no. 1-2, 10 May 2005 (2005-05-10), pages 83-89, XP027730530, ISSN: 0003-2670 [retrieved on 2005-05-10]
- WESCHLER C J ET AL: "Semivolatile organic compounds in indoor environments", ATMOSPHERIC ENVIRONMENT, PERGAMON, GB, vol. 42, no. 40, 1 December 2008 (2008-12-01), pages 9018-9040, XP025679812, ISSN: 1352-2310, DOI: 10.1016/J.ATMOSENV.2008.09.052 [retrieved on 2008-10-14]

## Description

### Field

The present invention relates to novel tracer substances for the analysis of semi-volatile organic contaminants (SVCs) and other toxic or noxious compounds present in the environment, such as PCBs (polychlorinated biphenyls), halogenated aliphatics, bisphenols and radon. The invention also describes methods for establishing multilayered sealant barriers towards SVCs which contain said tracer substances, as well as methods for assessing the integrity of said sealant barriers. The invention further provides curing coating compositions comprising such tracer compounds, and methods for their use. The invention finally relates to a coating composition kit of parts.

### Background of the Invention

Conditions for human habitation inside buildings can become challenged by the presence of both man-made and natural contaminants and other toxic or noxious compounds capable of permeating the outer structure of such buildings from the surrounding environment and/or being released from the building construction materials. Such compounds may over time pose a health risk to the occupants.

As an example of man-made toxic compounds present in the environment, **polychlorinated biphenyls** (PCBs) were used in many buildings in the 1950s up through the 1970s. PCBs were used as a plasticizer in caulk, added either during manufacture or mixed on site prior to installation. PCBs were also used in such different articles as plasticizers, coatings, inks, adhesives, and carbonless copy paper. Materials and components containing PCBs are still present today in many of these older buildings. PCBs are semi-volatile organic chemicals and can be transported in and around buildings through vaporization into the air and through absorption into dust and materials. PCBs can also diffuse through organic films such as paints and lacquers. PCBs may be present in the air, dust, soil and on surfaces in and around buildings leading to the potential for occupant exposure through multiple routes. The US Environmental Protection Agency's first assessment of PCB carcinogenicity was completed in 1987, and concluded that PCBs are probable human carcinogens.

The term "PCBs" cover any of 209 configurations of a biphenyl ring with various amounts of chlorine atoms substituted around each ring (2 to 10 possible Cl atoms), known as "PCB congeners". Approximately 130 of these congeners were commonly used in industrial applications. PCBs were widely used as dielectric and coolant fluids in items like electrical transformers, capacitors and electric motors.

These compounds were manufactured and sold in the United States under the trade name **Aroclor.** Each "aroclor" manufactured contains a distinctive subset of the 209 congener configurations. In the U.S., the most commonly used Aroclors were: 1221, 1232, 1242, 1248, 1254, and 1260. Aroclors are identified by number (such as 1248), with the last two digits representing the percent content of chlorine; higher Aroclor numbers reflect higher chlorine content. Testing for PCBs is often done by identifying and quantitating specific aroclors. However, testing for the specific congeners gives a much more detailed analysis of the PCB composition and at much lower concentrations.

**Chlorinated paraffins** (CPs) are part of the larger group of organic pollutants called halogenated aliphatics, and are normally divided into short- and medium chained chlorinated paraffins (SCCPs and MCCPs). They have a wide range of industrial applications, including their use in fillers or sealers, glues and coating materials for the building industry. SCCPs are classified as persistent, toxic and bioaccumulative (PTB) and are listed on the European Candidate list of substances of priority concern. There has been less attention on MCCPs, even though they have been found to have many similar properties as SCCPs. MCCPs are not yet restricted in the EU.

**Halogenated flame retardants** also belong to the class of halogenated aliphatics. One of the more notorious groups of flame retardants are hexabromocyclododecanes (HBCDDs), which have been used extensively in the building industry. HBCDDs are used in large quantities as flame retardants in building materials for thermal insulation made of expanded and extruded polystyrene (EPS/XPS). Like the CPs, hexabromocyclododecanes are lipophilic and persistent organic pollutants classified as persistent, toxic and bioaccumulative (PTB).

**Bisphenols** constitute yet another class of man-made toxic compounds present in the environment. They include **bisphenol A** (BPA), chemical name 4,4'-(Propan-2,2-diyl)diphenol, which is typically used as a chemical component of epoxy resins used in a wide range of building materials, including high performance coatings (paints, floor sealers, and other protective coatings), adhesives and fillers (caulk, grout, mortar, and putty), fiberglass binders, polycarbonate plastic and cement additives. Epoxy resins constitute nearly 30% of total bisphenol A (BPA) use. Epoxy resins are selected because of their corrosion protection, thermal stability and mechanical strength and are used primarily as coatings for a number of consumer and industrial applications. Another key use of epoxy resins is in construction materials.

BPA exhibits hormone-like (endocrine disruptor) properties, which has been linked to breast and prostate cancer which raise concern about its safety. As concerns over the potential health effects of BPA exposure have become publicized, manufacturers, consumers, and governments have taken steps to reduce BPA use and exposure. BPA has a low volatility and is primarily released into the environment through its use and handling.

Compounds such as the above and other xenobiotic compounds like polychlorinated dibenzo-p-dioxins (PCDD), polychlorinated dibenzofurans (PCDF), hexachlorobenzene (HCB), hexabromobenzene (HBB), 1,1-dichloro-2,2-bis(p-chlorophenyl)-ethene (4,4'-DDE) and the like are seen as an increasing health risk. These compounds have been classified as semi-volatile organic compounds (R.Rieger et al. Fresenius' J. Anal. Chem. 1995, Vol. 352, Issue 7-8, pp 715-724) and are furthermore relative stable towards hydrolytic or UV induced photolytic degradation, which means they are only slowly disappearing from the environment under normal conditions (according to the U.S. Environmental Protection Agency (EPA), a semivolatile organic compound is an organic compound which has a boiling point higher than water, and which may vaporize at temperatures above room temperature).

As an example of a naturally occurring toxic compound present in the environment, **radon** is a radioactive gas that results from the decay of uranium in rocks and soils. The gas is colourless, odourless and tasteless and can only be measured using special equipment. When radon surfaces in the open air, it is quickly diluted to harmless concentrations, but when it enters an enclosed space, such as a house or other building, it can sometimes build up to unacceptably high concentrations. Radon from the ground enters buildings chiefly through cracks and crevices in floors and cellar walls, or gaps around pipes or cables.

Radon has a molecular weight of 222 gr/mol and a density of 9.73 kg/m³ at standard temperature and pressure, i.e. about 8 times the density of the Earth's atmosphere at sea level. Due to its high density, radon is not easily dissipated from enclosed areas, and is in this respect functionally quite similar to the organic contaminants mentioned above.

Radon decays to form tiny radioactive particles, some of which stay suspended in the air. When these particles are inhaled into the lungs, they give a radiation dose that may damage cells in the lung. Radon has been shown to be a cause of cancer, specifically lung cancer; prolonged exposure to elevated levels of radon gas in an enclosed area can be a contributory factor in increasing the risk of lung cancer, particularly where other factors such as cigarette smoking are involved.

It is often desirable to form a barrier on building surfaces in order to prevent semi-volatile contaminants like the ones mentioned above (in the following referred to as SVCs), including radon, from entering the building interior. This can be achieved by applying multiple coats of a curing (i.e. hardening) sealing composition to the surface, thereby forming an impenetrable multi-layer barrier to the compounds in question.

Sealant compositions of various types have been developed which effectively block the migration of SVCs through and/or from different types of construction materials as tested under controlled conditions, i.e. when the surfaces to be treated are clean, dry and substantially planar and display limited dimensional variability under changing conditions of temperature and/or humidity.

After such sealing compositions have been applied and allowed to cure under realistic conditions "in the field", the question may arise, however, whether the resulting barrier will be durable. Even when control experiments in a laboratory environment have concluded that the desired imperviousness can be achieved when a barrier having the prescribed layer thickness has been formed on the surface, it is not always possible under realistic conditions to achieve either a perfect adherence to the surface, or a homogeneous layer thickness for the applied sealing composition.

Further problems may even arise, because the building materials forming the surface to be coated typically expand or contract following annual or even diurnal variations in temperature and humidity. Such a dimensional variability may not be compatible with the cured coating composition, and may lead to cracks in the applied barrier. Cracks in walls, ceiling or floors are moreover often caused by the settling or shifting of the building construction.

These various causes may eventually lead to invisible micro-cracks and crevices in the barrier and loss of adherence to the surface. In situations like these, where the integrity of the initially tight barrier has been compromised, there is a real risk that the SVCs for which the barrier was put in place may now penetrate the barrier through the cracks and crevices formed over time, even in the absence of visible faults in the barrier.

When checks are performed of the air inside buildings which have previously had issues with SVCs permeating the building surfaces, or escaping from such surfaces, it is therefore not unusual to find the same SVCs once again present in the air after some time has passed.

It is however not always the reappearance of a known SVC is caused by a faulty barrier; sometimes a new source of the known SVC has just become active. Unfortunately, in such cases it may prove difficult to establish with certainty whether the SVC source is new or old, which may lead to unnecessary repairs of intact barriers.

There is thus a need for a reliable method for establishing whether the presence of a specific SVC in the surrounding air is caused by a new contaminant source, or by a fault in an established barrier, and if so, in which part of the barrier, such that repair costs can be minimized and potential new contaminant sources be sealed up.

### Prior art

The following prior art was cited in the international (PCT) and regional (EP) phase:
1. GB 2 462 668 A
2. WO 2013/079521 A1
3. KUBWABO C ET AL: "Quantitative determination of free and total bisphenol A in human urine using labeled BPA glucuronide and isotope dilution mass spectrometry", ANALYTICAL AND BIO- ANALYTICAL CHEMISTRY, SPRINGER, DE, vol. 406, no. 18, 1 July 2014, pages 4381 4392.
4. KAWAGUCHI MET AL: "Molecularly imprinted solid phase extraction using stable isotope labeled compounds as template and liquid chromatography-mass spectrometry for trace analysis of bisphenol A in water sample", ANALYTICA CHIMICA ACTA, ELSEVIER, AMSTERDAM, NL, vol. 539, no. 1-2, 10 May 2005, pages 83-89.
5. US 2011/027591 A1

**D1** is a patent application which is directed towards a coating composition (e.g. a glaze composition for protecting vehiclepaintwork) comprising a UV tracer, said composition being adapted to provide a coating which fluoresces when exposed to UV electromagnetic radiation. Coverage of the coating composition on a substrate can be assessed using the tracer, which may be an optical brightener such as a benzoxazole compound.

**D2** is a patent application which relates to the field of authentication of coating compositions such as varnishes, inks and paints, and it is particularly useful in the field of authentication of such coating compositions when applied to substrates like banknotes or other valuable documents. It is particularly directed to a marked coating composition, e.g. an ink, that is marked with a marker (taggant) such as to allow for its authentication, and a method for authenticating such a marked coating composition. The marking is achieved by covalently binding a taggant to a coating composition component, e.g. such used for security documents. The taggant, which is not extractable through usual chemical treatments like washing methods or the use of solvents, can be detected and identified upon thermally induced chemical fragmentation with a PY-GC-MS apparatus. The marked ink or the marked substrate is thus authenticated as belonging to a particularly marked ink, varnish or batch.

**D3** is a scientific paper describing the development of a sensitive and improved method for the measurement of free and total BPA in human urine. Use of a labeled conjugated BPA (bisphenol A-d6 β-D-glucuronide) allowed for the optimization of the enzymatic reaction and permitted an accurate determination of the conjugated BPA concentration in urine samples.

**D4** is a scientific paper describing the development of a molecularly imprinted polymer (MIP) using a stable isotope labeled compound as the template molecule, called the "isotope molecularly imprinted polymer" (IMIP). In the study, bisphenol A (BPA) was used as the model compound. None imprinted polymer (NIP), MIP, dummy molecularly imprinted polymer (DMIP) and IMIP were prepared by the suspension polymerization method using without template, BPA, 4-tert-butylphenol (BP) and bisphenol A-d16 (BPA-*d*₁₆), respectively.

**D5** is a patent application which relates to two-component or multicomponent aqueous epoxy resin primer compositions comprising a first component, a second component and an optional other component. The first component contains at least water and an epoxy resin while the second component contains at least one polyamine. The composition also comprises carbon black and/or epoxy silane and/or epoxy siloxane and/or an amino silane and/or an amino siloxane and/or mercaptosilane. The aqueous epoxy resin primer compositions improve adhesion of moisture-curing polyurethane adhesives or sealants of various bases.

### Definitions

As used herein, the term "tracer substance" shall refer to an organic compound, preferably a semivolatile organic compound, which can be used in the analysis for semi-volatile contaminants (SVCs) in the environment which have diffused through or permeated a multilayer barrier meant to contain the SVC, wherein the tracer substance has been added to the bottom (or first) layer of the barrier. To be useful as a tracer substance, it should act in the same way as the SVC in question as regards diffusion and dissipation characteristics, and may have some structural features in common with the SVC. Moreover, a tracer substance according to the present invention contains one or more marker groups which render it easy to analyse for, using for example mass spectrometry.

As used herein, the term "specific tracer substance" shall refer to a tracer substance which is structurally closely related to a specific SVC, ie. has a substantial structural resemblance to the SVC in question.

As used herein, the term "semi-volatile contaminants", abbreviated "SVCs" refers ¹⁾ to organic contaminants and other toxic or noxious compounds present in the environment having a boiling point higher than water, and which may vaporize when exposed to temperatures above room temperature (in accordance with the U.S. Environmental Protection Agency (EPA) definition of semivolatile organic compounds, SVOCs), and ²⁾ to the naturally ocurring radioactive gas Radon, which due to its very high density acts similarly to SVOCs when trapped inside buildings, and further share the ability to permeate constructions via cracks and crevices. SVCs according to the present invention include polychlorinated biphenyls (PCBs), Chlorinated paraffins (CPs), Halogenated flame retardants, Bisphenols and other xenobiotic compounds like polychlorinated dibenzo-p-dioxins (PCDD), polychlorinated dibenzofurans (PCDF), hexachlorobenzene (HCB), hexabromobenzene (HBB), 1,1-dichloro-2,2-bis(p-chlorophenyl)-ethene (4,4'-DDE), and the like.

As used herein, the term "diffusion" refers to the process when molecules from a material - typically in a gaseous state - move from an area of high concentration (where there are lots of molecules) to an area of low concentration (where there are fewer molecules). Molecules tend to move from places of high concentration to places of low concentration, just by moving randomly, in accordance with Le Chatelier's principle.

As used herein, the term "dissipation" refers to the the scattering or dispersal of vaporized compounds. The dissipation behaviour of a specific compound thus depends *i.a.* on the enthalpy of vaporization and the density of the resulting gas, but also on the propensity of the compound to cling to surfaces.

As used herein, the term "semivolatile organic compounds", abbreviated SVOCs, refers to organic molecules that can be abundant in both the gas phase and condensed phase, represented by vapor pressures between 10⁻¹⁴ and 10⁻⁴ atm (10⁻⁹ to 10 Pa) [see eg. Weschler et al., Semivolatile organic compounds in indoor environments. Atmospheric Environment. 2008, 42, 9018-9040]. Because of their slow rate of release from sources and because of their propensity to partition into sorbed states, SVOCs can persist for years indoors, akin to persistent organic pollutants in the outdoor environment (POPs).

As used herein, the term "marker group" shall refer to a chemical functional group which is part of a tracer substance, and which makes the tracer substance produce ion fragments in the mass spectrometer that are unusual in nature because the marker group contains elements like fluorine and deuterium, which are not found in abundance in nature in general, and are extremely rarely found in construction materials. As examples of such marker groups can be mentioned the deutero (D), fluoro (F), CF₃ (trifluoromethyl) group and the CD₃ (trideuteromethyl) group. Derived alkoxy groups like OCD₃ and OCH₂CF₃ (trideuteromethoxy and 1,1,1-trifluoroethoxy) are for the same reason also excellent marker groups.

As used herein, the term "multilayered barrier" shall refer to two or more coats of a hardened (i.e. cured) sealing composition applied to a surface; typically a silica based surface like concrete or other cementitious surfaces, which effectively block the migration of SVCs and thereby act as a barrier. A multilayered barrier is typically constructed by letting one layer, or coat, of a curing sealing composition cure before applying the next layer. One or more primer layers may be applied before the first layer of sealing composition, especially for porous surfaces.

### STATEMENTS OF INVENTION

According to the present invention the integrity of a multilayered barrier towards a specific SVC can be assessed by analyzing for a **specific tracer substance** which has been added to the first coat of a curing sealing composition. The specific tracer substance must fulfill certain requirements:
- It must be chemically stable so it is not degraded in the sealing composition.
- It should have diffusion properties which are similar to those of the SVC in question, and should further be structurally similar to the SVC in question. This ensures that the SVC and the tracer substance will permeate the barrier layers at similar velocities.
- It should have a boiling point similar to that of the SVC in question. This ensures that the tracer will be present in the air and on the surface of the barrier in a ratio matching that of the SVC in question.
- It must have a chemical composition which renders it easy to identify by suitable analytical methods.
- It should preferably be a compound which is itself very rare in nature, or contain chemical moieties which are very rarely found in nature.

The co-presence in the air inside a building of a particular SVC together with an associated specific tracer substance which fulfills the above criteria will thus indicate a migration through the barrier due to one or more of the above-mentioned causes, such as cracks having developed in the barrier, which again indicates that the SVC is no longer fully contained by the barrier. Due to the similarities in diffusion and dissipation properties, the level found for the tracer will indicate how fast the SVC presently permeates the barrier.

Conversely, if the relevant SVC is detected inside a building but no sign of the associated specific tracer substance can be found, then this strongly points to a new source of the SVC which has not yet been sealed up, and not to a compromised barrier.

The present invention also provides the use of a non-specific, or **generic tracer substance,** which does not have a substantial structural resemblance with a specific SVC, but retains a number of the characteristics of the specific tracer substance and is used in the same way, ie being added to the to the first coat of a curing, multi-layer sealing composition.
- It must be chemically stable so it is not degraded in the sealing composition.
- It should be a semi-volatile compound.
- It should be easy to identify by suitable analytical methods.
- It should preferably be a compound which is itself very rare in nature, or contain chemical moieties which are very rarely found in nature.

The use of a generic tracer substance does not allow for a precise assessment of the rate of dissipation of a given SVC, but will by its presence in the air be a telltale signal that cracks have developed in the barrier which may allow SVCs like eg. radon to pass through the barrier. A generic tracer according to the present invention may have the advantage of being easier to analyze for than a particular SVC like eg. radon.

According to the present invention, the presence of cracks and crevices in a multilayered barrier can be thus recognized by analyzing for a generic tracer substance which has been added to the first layer of a curing sealing composition, and which fulfill the above mentioned requirements.

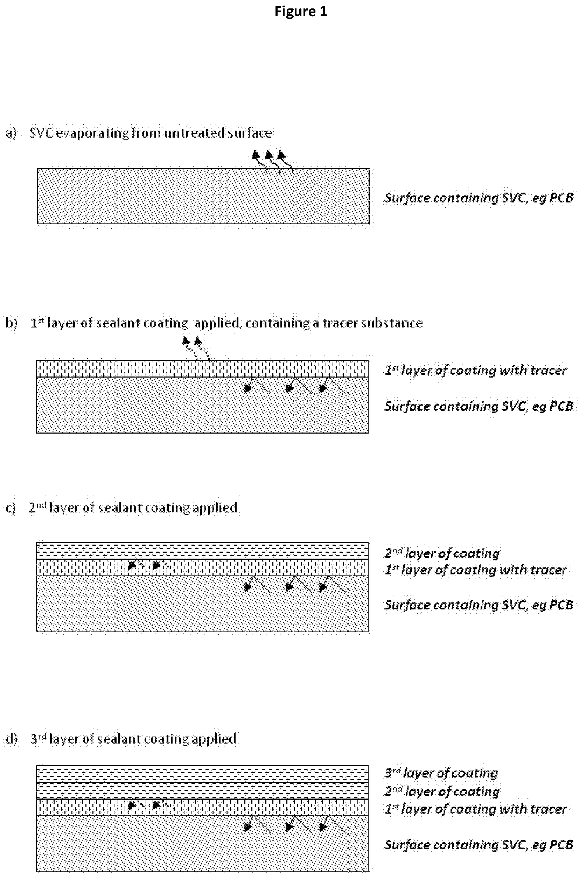

The general concept of the invention is illustrated in Figure 1-3.

Specific tracer substances according to the present invention should be selected from semi-volatile organic compounds, and in a given situation should be chosen to mimic as closely as possible the dissipation and diffusion behaviour of the SVC(s). A prediction of the behaviour of a new tracer substance can be based on the similarity of the Hansen Parameters, and experimental verification can be obtained by size exclusion chromatography.

In order to be able to detect very low levels of the tracer compounds, it is preferred to use detection by mass spectroscopy (MS), typically coupled with gas chromatography (GC-MS). It is moreover preferable if the tracer substance produces ion fragments in the mass spectrometer that are unusual in nature. Fluorine is a rare constituent in organic compounds in nature, and therefore a fluorine atom or a CF₃ group is very unlikely to be found in ordinary construction materials. The mass is unusual and the isotope pattern unique. The deuterium atom and the perdeuterated methyl group, CD₃, are also highly unusual because deuterium (or "heavy hydrogen") is only present in amounts of ∼0.01% of naturally occurring hydrogen. A deuterium atom or a CD₃ group are therefore also very unlikely to be found in ordinary construction materials.

Neither deuterium nor fluorine influences the boiling point of organic compounds to a large degree. For example, benzene has a bp of 80 °C; monofluorobenzene a bp of 85 °C; 1,2-difluorobenzene a bp of 92 °C; 1,3 difluorobenzene a bp of 83 °C and 1,4 difluorobenzene a bp of 89 °C. 1,3,5-trifluorobenzene and hexafluorobenzene both have lower boiling points than the difluorobenzenes. The same tendency holds true for aliphatic compounds (Tamejiro Hiyama: Organofluorine Compounds: Chemistry and Applications, Springer Verlag 2013). For the deuterated compounds, monodeuterobenzene has a bp of 80 °C (as normal benzene) whereas hexadeuterobenzene has a bp of 79 °C.

This almost neglible effect on boiling point renders the fluoro and deutero group ideal candidates for developing tracer compounds according to the present invention. Fluoro- or deutero-labeled compounds will typically have practically the same boiling point as their unlabeled analogues - and therefore very similar volatilities - but due to their specific and "unnatural" mass are easily detectable by mass spectrometry.

These two chemical groups, CF₃ and CD₃, are thus examples of marker groups that can be detected at the ppb level in a sample. Inclusion of either or both these groups in a tracer substance renders the tracer substance easy to identify by mass spectrometry, as the spectrometer can be set up to detect only these fragments. This simplifies the analysis and raises the sensitivity by a significant factor.

The CF₃ group can be introduced in other organic compounds through a variety of chemical reactions, and in both aromatic and non-aromatic compounds through radical trifluoromethylation, electrophilic or nucleophilic trifluoromethylation, by using commercially available reagents such as trifluoromethyltrimethylsilane, sodium trifluoroacetate, trifluoromethane, trifluoroiodomethane, trifluoromethyl sulfone, trifluoromethanesulfonyl chloride and sodium trifluoromethanesulfinate. The CF₃ group can also be introduced as part of a larger functional group, such as the trifluoroethoxy group by employing a metal salt of trifluoroethanol such as sodium trifluoroethoxide (CF₃CH₂ONa) as a nucleophile.

The CD₃ group can also be introduced in other organic compounds through a variety of chemical reactions including many reactions similar to those mentioned above for the CF₃ group, but also by the use of *d₆*-acetone ((CD₃)₂C=O) as reagent in condensation reactions, or by employing deuterochloroform (CDCl₃) in electrophilic coupling reactions.

Deuterium can also be introduced through exchange reactions where hydrogen or, in some cases, halogen atoms present in the molecule are replaced by deuterium in the presence of a catalyst. Deuterium gas (D₂) and deuterated water (D₂O, "heavy water") are commonly used in such reactions. Deuterium can also be introduced by reduction reactions employing eg. lithium aluminium deuteride (LiAlD₄) or sodium borodeuteride (NaBD₄).

Apart from ensuring an easy identification of the tracer compound, it is also an object of the present invention to provide specific tracer substance which mimic the dissipation and diffusion behaviour of the relevant SVC(s) such that for example the rate of release can be assessed. This is ensured by attaching relevant marker groups such as F, D, CF₃ and/or CD₃ to organic molecules which bear substantial structural resemblance to the SVCs in question. The resulting compound will thus typically behave quite similarly to the SVC as regards eg. ease of vaporization and diffusion through construction materials, and due to the attached marker group will be easily identifiable as a tracer compound.

The skilled person wishing to produce a tracer substance for a specific SVC in accordance with the present invention will thus typically aim for a compound which is structurally very similar to the SVC in question, but which contains one or more marker groups which renders the tracer molecule easy to identify in very low concentrations.

The choice of tracer substance will therefore depend on the specific SVC and the circumstances under which the analysis for re-emergence of the SVC into the environment will be carried out. If the analysis is to be carried out by chromatography, such as GC, it is preferable to select a tracer substance with a different retention time than the SVC to avoid overlap in the eventual chromatogram. It may thus be necessary to test more than one tracer substance to find the ideal tracer substance for a given SVC, but this is done by routine testing and does not require any inventive skills.

The choice of tracer substance will also depend on the availability of suitable starting materials which can be used for introducing one or more marker groups as described above.

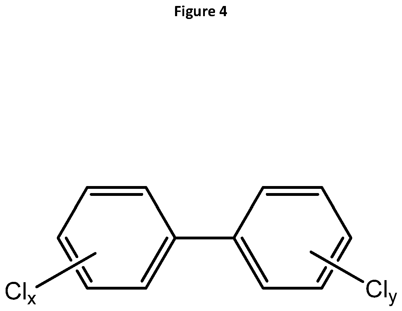

As a non-limiting example of the present invention can be mentioned developing suitable tracer substances for **polychlorinated biphenyls,** PCBs, which are compounds of the general structure shown in **Fig 4** wherein x+y = 1-10. PCBs are a family of 209 congeners (according to the IUPAC definition, "congeners" are chemical substances which are related to each other by origin, structure, or function). Unless specifically synthesized or purified, an industrially produced quantity of "PCB" is by default a blend of multiple molecule types because each molecule forms independently, and chlorine does not strongly select which site(s) it bonds to.

PCBs which contain between 2-6 chlorine atoms are amongst the most commonly used, and have molecular weights of app. 220-360.

A specific tracer for PCBs should contain the biphenyl moiety, as this is common for all PCBs. However, as chlorine has two major (stable) isotopes, i.e. ³⁵Cl and ³⁷Cl (in a ^{∼}3:1 ratio), a potential tracer substance containing chlorine will give rise to two peaks in a mass spectrum, with two mass units in between. This will lower the sensitivity somewhat of the analysis, and therefore a tracer substance containing no chlorine atoms would be preferable.

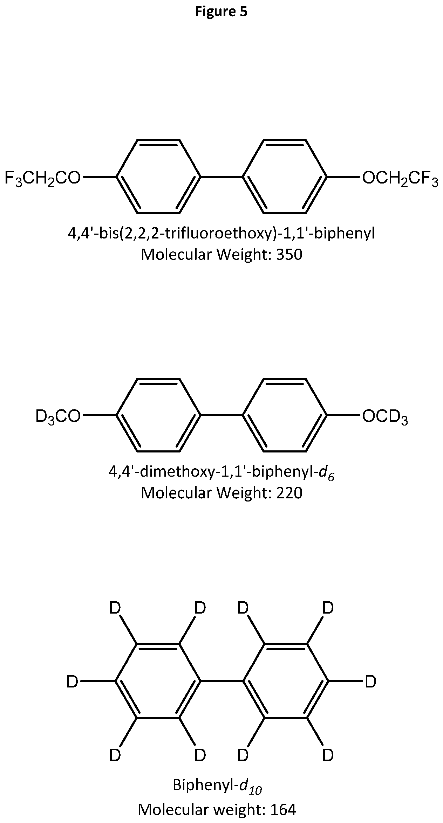

As mentioned above the CD₃ and CF₃ groups are excellent marker groups, and accordingly tracer substances containing these two groups were devised: 4,4'-bis(2,2,2-trifluoroethoxy)-1,1'-biphenyl containing two trifluoroethoxy groups and a molecular weight of 350, and 4,4'-dimethoxy-1,1'-biphenyl-*d*₆, containing two trideuteromethoxy groups and a molecular weight of 220 as shown in **Fig 5****.** In terms of molecular weight these two compounds thus cover a substantial section of commercially interesting PCBs.

As the most commonly used PCBs have boiling points in the range of 285°C (monochlorobiphenyl) to app 400° C (hexachlorobiphenyl), and typically have been employed as a mixture of individual congeners, it is important to choose the one or more tracer substances such that their volatility matches the PCB congeners most likely to be present. The PCB composition of relevance in a specific situation can be verified by analysis before the PCB contamination is covered by a coating composition according to the present invention.

The two compounds in **Fig 5** can be produced from 4,4'-dibromo-1,1'-biphenyl by reaction with the sodium salt of trifluoroethanol (sodium trifluoroethoxide) in the presence of a copper salt, and from 4,4'-dihydroxy-1,1'-biphenyl by alkylation with methyl iodide-d3, respectively.

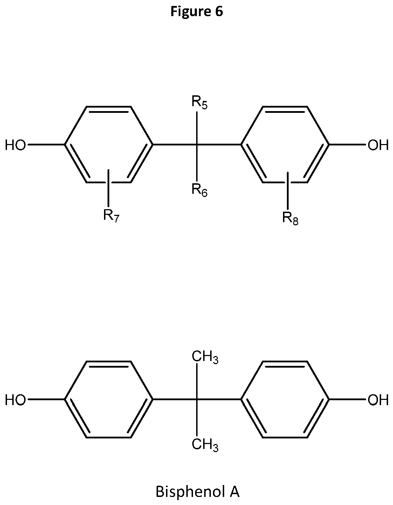

As another non-limiting example of the present invention can be mentioned suitable tracers for **bisphenols.** In the context of this application, bisphenols are compounds of the general structure shown in **Fig 6** wherein R₅ and R₆ can be alkyl or phenyl, and R7 and R8 can be hydrogen or alkyl. As an example can be mentioned **bisphenol A** (BPA), wherein R₅ = R₆ = methyl and R₇ = R₈ = hydrogen.

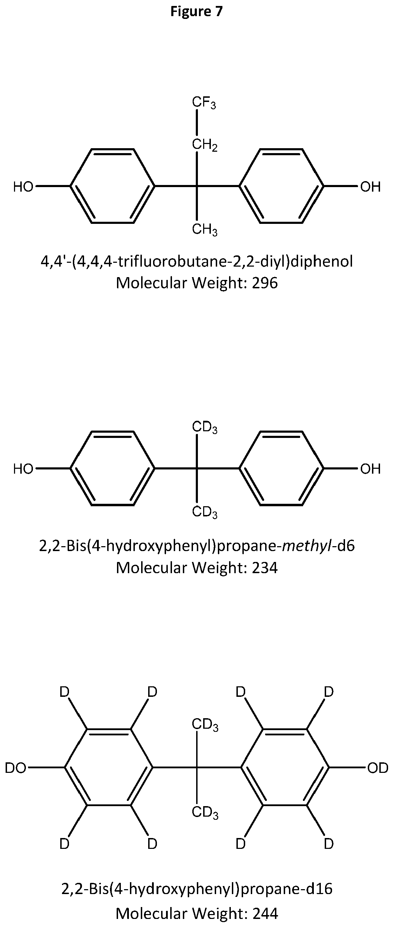

As mentioned above the CD₃ and CF₃ group are excellent marker groups, and accordingly the tracer substances 4,4'-(4,4,4-trifluorobutane-2,2-diyl)diphenol; 2,2-bis(4-hydroxyphenyl) propane-*d₁₆* and 2,2-bis(4-hydroxyphenyl) propane-*methyl*-*d₆* shown in **Fig 7** were devised. These compounds can be synthesized under acidic conditions by condensation of phenol with either trifluoroethylmethylketone or hexadeutero acetone, respectively, and ring-substituted analogous compounds wherein R₇ and R₈ are not hydrogen can be synthesized from substituted phenols. Some of these compounds are also commercially available.

The present description thus provides ample guidance for the preparation of relevant tracer substance for a range of common SVCs, which will allow the skilled person to establish a suitable tracer substance to use in any given situation. In the following the various aspects of the invention will be described.

In a first aspect of the invention a tracer substance for SVCs is selected from compounds according to the following Formula I: 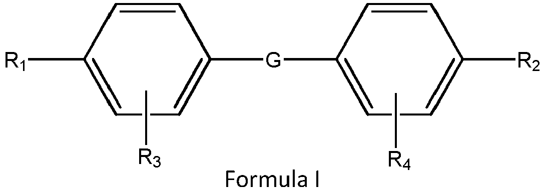 wherein
R₁ is selected from hydrogen, deuterium, hydroxy, C₁-C₆ alkoxy, trifluoromethoxy, 1,1,1-trifluoroethoxy or 1,1,1-trifluoropropoxy, and
R₂ is selected from hydrogen, deuterium, hydroxy, C₁-C₆ alkoxy, trifluoromethoxy, 1,1,1-trifluoroethoxy or 1,1,1-trifluoropropoxy, and
R₃ is selected from hydrogen, deuterium, fluorine, C₁-C₆ alkyl, trifluoromethyl or trifluoromethoxy, and
R₄ is selected from hydrogen, deuterium, fluorine, C₁-C₆ alkyl, trifluoromethyl or trifluoromethoxy,
and wherein
the linker group G may be absent, ie the two aromatic rings are directly linked by a single bond, or
the linker group G is a carbon atom linked to 1 or 2 C₁-C₆ alkyl groups optionally substituted with up to 6 fluorine atoms and/or up to 6 deuterium atoms,
and wherein the tracer substance contains at least one deuterium atom and/or one fluorine atom.

In **a second aspect** of the invention there is provided a coating composition comprising the following three components:
1. an epoxysiloxane of formula

   R₁-Si(OR₂)₃

   wherein
   R₁ = a C₅ to C₃₀ alkyl group containing one or more epoxy groups,
   R₂ = a C₁ to C₆ alkyl group, and
2. an aminosiloxane of formula

   R₃-Si(OR₄)₃

   wherein
   R₃ = a C₅ to C₃₀ alkyl group containing one or more amino groups,
   R₄ = a C₁ to C₆ alkyl group, and
3. a compound of Formula I according to the first aspect of the present invention.

In a **third aspect** of the invention there is provided a process for treating surfaces comprising the steps of:
a. optionally applying one or more layers of a primer;
b. forming a first barrier over a surface, the first barrier comprising a first coating composition, a tracer substance according to the first aspect of the invention, and optionally having a first color;
c. once the first barrier has cured, forming a second barrier over the first barrier, the second barrier comprising a second coating composition, and optionally having a second color, and;
d. once the second barrier has cured, forming a third barrier over the second barrier, the third barrier comprising a third coating composition, and optionally having a third color.

In a **fourth aspect** there is provided a multi-layered barrier covering a surface, comprising two or more cured or hardened layers of a coating composition, wherein the first applied layer of coating composition contains a tracer substance according to the first aspect of the invention.

In a **fifth aspect** of the invention there is provided a coating composition kit, comprising the following parts:
a) Component 1 containing a tracer substance according to the first aspect of the invention,
b) Component 1 without tracer substance,
c) Component 2, and
d) a set of instructions for mixing Component 1 and 2, and for carrying out the surface treating process according to the second aspect of the invention.

In a **sixth aspect** there is provided an analytical method for field testing surfaces covered by a multi-layered barrier according to the fourth aspect of the present invention, in order to determine the co-presence of an SVC (such as a PCB) and a tracer substance according to the first aspect of the invention, using automated test equipment including a suitable gas chromatographic (GC) column connected to a mass spectrometric (MS) detector and a microprocessor including a memory in which is located data regarding magnitudes and retention times for a plurality of standard SVC mixtures and tracer substances, said method comprising the following steps:
a. preparing a sample by using a High Volume Sampler (HVS) for SVCs in ambient air, or a standardized wipe sample for surfaces potentially contaminated with SVCs;
b. separating and analyzing the components in said sample on a said gas chromatographic column using said MS detector which has an input for receiving said sample and an output for providing separated component peaks;
c. providing an electrical output signal including information regarding the magnitude and retention time of component peaks using said mass spectrometric detector which is connected to the output of said GC column;
d. comparing said electrical output with the contents of said memory through the use of said microprocessor; and
e. indicating one of the following:
   I. the presence of an SVC in said analytical sample based upon comparison with data in said microprocessor memory,
   II. the co-presence of an SVC in said analytical sample together with said tracer substance or,
   III. no SVC or tracer is present in the analytical sample,
   wherein the co-presence of a SVC together with the tracer substance (option e-II) is indicative of cracks or other faults in the multi-layered barrier, whereas the presence of an SVC alone in the analytical sample indicates a new source of the SVC.

### FIGURES

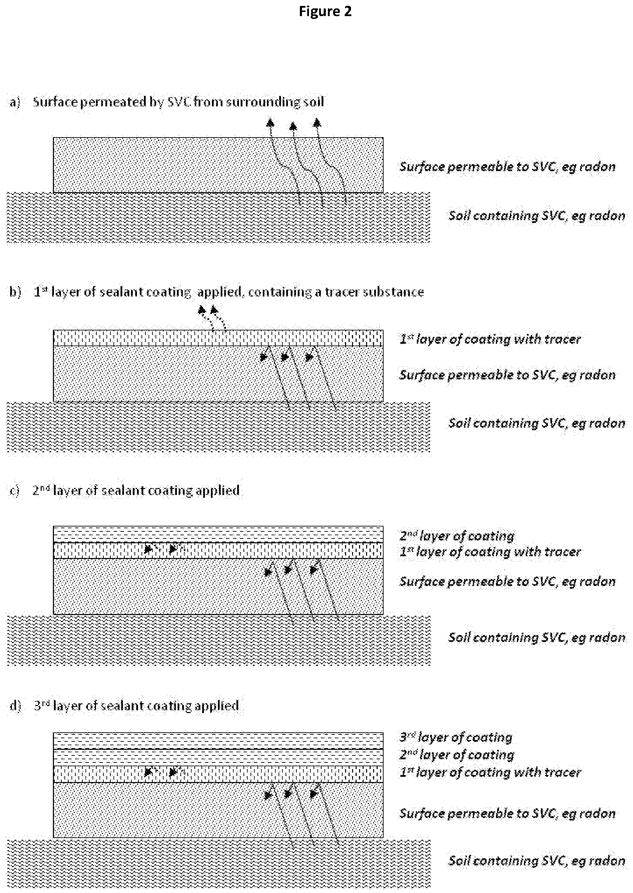

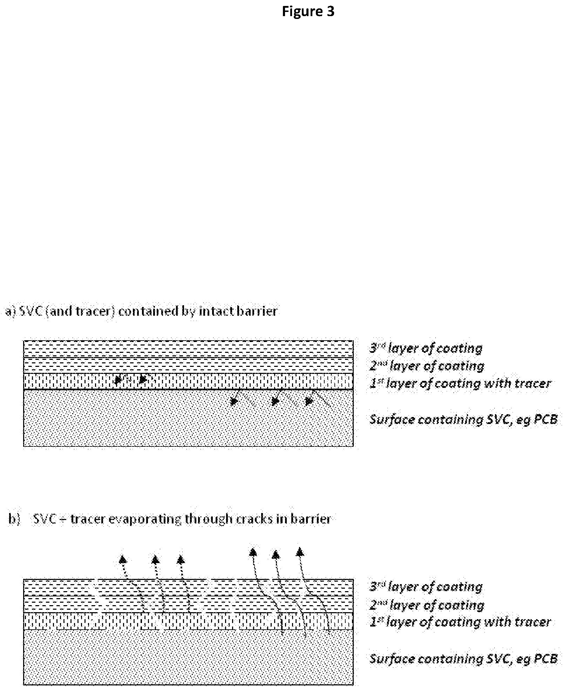

Figure 1:
   a) shows a sideway view of a contaminated surface from which a SVC like eg. PCB evaporates (wavy arrows).
   b) the same surface as in Fig.1 a) with a first layer of coating applied, which contains a tracer substance. This layer of coating blocks the evaporation of the SVC. The added tracer substance evaporates from the surface of the first layer of coating (wavy, dotted arrows).
   c) the same surface as in Fig.1 b) with a second layer of coating applied. This layer of coating blocks the evaporation of the tracer substance and provides additional protection against evaporation of the SVC (angular reflected arrows).
   d) the same surface as in Fig.1 c) with a third layer of coating applied. This layer of coating provides additional protection against evaporation of the SVC.
Figure 2:
   a) shows a sideway view of a surface permeated by a SVC from the surrounding soil, eg radon (wavy arrows).
   b) the same surface as in Fig.2 a) with a first layer of coating applied, which contains a tracer substance. This layer of coating blocks the permeation of the SVC (angular reflected arrows). The added tracer substance evaporates from the surface of the first layer of coating (wavy, dotted arrows).
   c) the same surface as in Fig.2 b) with a second layer of coating applied. This layer of coating blocks the evaporation of the tracer substance and provides additional protection against permeation of the SVC.
   d) the same surface as in Fig.2 c) with a third layer of coating applied. This layer of coating provides additional protection against permeation of the SVC.
Figure 3:
   a) shows a sideway view of a contaminated surface with three layers of coating applied, including a first layer of coating containing a tracer substance. The resulting barrier is intact, and protects against evaporation of the SVC.
   b)
   the same surface as in Fig.3 a) wherein cracks have developed in the barrier over time. Both the SVC and the tracer substance evaporate (wavy arrows, both solid and dotted).
Figure 4:
   General formula for polychlorinated biphenyls.
Figure 5:
   Three exemplary tracer substance for PCBs: 4,4'-bis(2,2,2-trifluoroethoxy)-1,1'-biphenyl, 4,4'-dimethoxy-1,1'-biphenyl-*d₆* and biphenyl-*d₁₀*
Figure 6:
   General formula for bisphenols and the specific structure for bisphenol A.
Figure 7:
   Three exemplary tracer substance for bisphenols: 4,4'-(4,4,4-trifluorobutane-2,2-diyl)diphenol; 2,2-bis(4-hydroxyphenyl) propane-*d₁₆* and 2,2-bis(4-hydroxyphenyl) propane-*methyl-d₆.*
Figure 8:
   Experimental set-up for example 4.1 with concrete bricks having different areas sealed.
Figure 9:
   Stainless steelbox with attached sampling tube for air sampling
Figure 10:
   Graphs showing relation between the available relative area for evaporation and the air concentration of PCBs and the tracer substance 4,4'-2,2,2-trifluoroethoxy biphenyl
Figure 11:
   Experimental set-up for example 4.2 with concrete bricks having different areas sealed.
Figure 12:
   Experimental set-up for example 4.3 with concrete bricks having different areas sealed.

### DETAILED DESCRIPTION OF THE INVENTION

In a **first aspect** of the invention a tracer substance is selected from compounds according to the following Formula I: 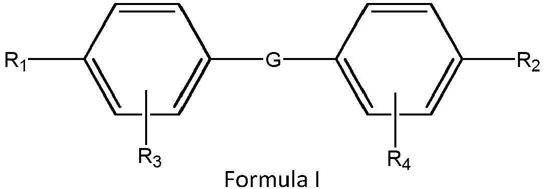 wherein
R₁ is selected from hydrogen, deuterium, hydroxy, C₁-C₆ alkoxy, trifluoromethoxy, 1,1,1-trifluoroethoxy or 1,1,1-trifluoropropoxy, and
R₂ is selected from hydrogen, deuterium, hydroxy, C₁-C₆ alkoxy, trifluoromethoxy, 1,1,1-trifluoroethoxy or 1,1,1-trifluoropropoxy, and
R₃ is selected from hydrogen, deuterium, fluorine, C₁-C₆ alkyl, trifluoromethyl or trifluoromethoxy, and
R₄ is selected from hydrogen, deuterium, fluorine, C₁-C₆ alkyl, trifluoromethyl or trifluoromethoxy,
and wherein
the linker group G may be absent, ie the two aromatic rings are directly linked by a single bond, or
the linker group G is a carbon atom linked to 1 or 2 C₁-C₆ alkyl groups optionally substituted with up to 6 fluorine atoms and/or up to 6 deuterium atoms,
and wherein the tracer substance contains at least one deuterium atom and/or one fluorine atom.

In Formula I the open carbon atom positions are numbered 2 to 6 in the left ring and 2' to 6' in the right ring. The positions 2, 2', 6 and 6' are also called ortho-positions (o-); 3, 3', 5 and 5' are also called *meta*-positions (*m-*), and 4 and 4' are called para-positions (p-).

In a preferred embodiment of the first aspect of the invention the compounds according to Formula I wherein the linker group G is absent, are particularly well-suited as tracer substance for polychlorinated biphenyls (PCBs).

In a particular embodiment there is provided the use of a compound of Formula I as a tracer substance in sealing compositions for PCB-containing surfaces.

The following positional isomers are further particularly preferred embodiments of the first aspect of the invention wherein the linker group G is absent:

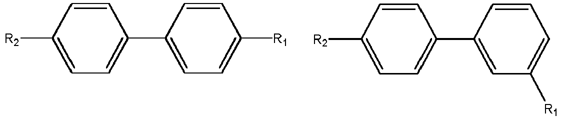

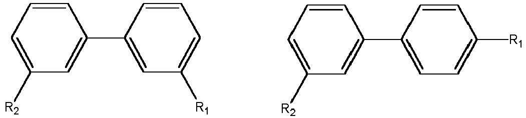

Even though the compounds of Formula I wherein the linker group G is absent are preferably employed as specific tracer substances for PCBs, they can also be used as tracer substance for other natural or man-made contaminants SVCs according to the present invention, such as chlorinated dibenzofurans and benzodioxines having similar diffusion properties and boiling points.

In a preferred embodiment the compound of Formula 1 contains at least one fluorine atom.

In another preferred embodiment the compound of Formula 1 contains at least one deuterium atom.

In another preferred embodiment the compound of Formula 1 contains at least one trifluoromethyl (-CF₃), trifluoromethoxy (-OCF₃) or trifluoroethoxy (-OCH₂CF₃) group.

In another preferred embodiment the compound of Formula 1 contains at least one trideuteromethyl (-CD₃) group.

In a further preferred embodiment the linker group G is absent, R₃ and R₄ are both hydrogen and R₁ and R₂ are both 1,1,1-trifluoroethoxy placed in the 3, 3', 4 or 4' positions.

In a particularly preferred embodiment, the compound of Formula 1 is 4,4'-bis(2,2,2-trifluoroethoxy)-1,1'-biphenyl, ie R₁ = R₂ = CF₃CH₂O-, where R₁ and R₂ are placed in the 4'- and 4 - positions of the compound of Formula I, respectively.

In a particular embodiment there is provided the use of 4,4'-bis(2,2,2-trifluoroethoxy)-1,1'-biphenyl, ie R₁ = R₂ = CF₃CH₂O-, where R₁ and R₂ are placed in the 4'- and 4 - positions of the compound of Formula I, respectively, as a tracer in sealing compositions for PCB-containing surfaces.

In another preferred embodiment of the first aspect of the invention the compounds according to Formula I wherein the linker group G is a carbon atom linked to 1 or 2 C₁-C₆ alkyl groups optionally substituted with up to 6 fluorine atoms and/or up to 6 deuterium atoms, are particularly well-suited as tracer substances for bisphenols, such as bisphenol A.

The following positional isomers are further particularly preferred embodiments of the first aspect of the invention wherein the linker group G is is a carbon atom linked to 1 or 2 C₁-C₆ alkyl groups optionally substituted with up to 6 fluorine atoms and/or up to 6 deuterium atoms and R₁, R₂, R₃ and R₄ remain defined as above mentioned:

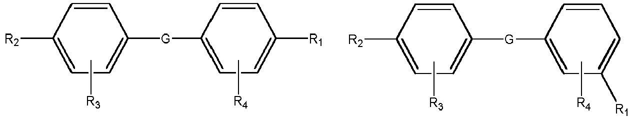

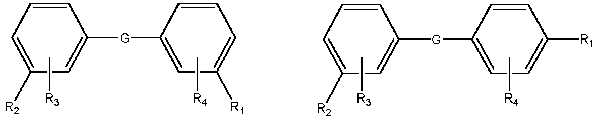

Even though the compounds of Formula I wherein the linker group G is is a carbon atom linked to 1 or 2 C₁-C₆ alkyl groups optionally substituted with up to 6 fluorine atoms and/or up to 6 deuterium atoms are preferably employed as tracer substance for bisphenols such as bisphenol A, they can also be used as tracer substance for other natural or man-made contaminants SVCs according to the present invention, such as chlorinated dibenzofurans and benzodioxines having similar diffusion properties and boiling points.

In a further preferred embodiment the compounds of Formula I, wherein the linker group G is a carbon atom linked to 1 or 2 C₁-C₆ alkyl groups optionally substituted with up to 6 fluorine atoms and/or up to 6 deuterium atoms, R₁, R₂, R₃ and R₄ are all hydrogen and the compound of Formula I contain at least one CD₃ and/or at least one CF₃ group, such as one CD₃ group, two CD₃ groups, one CF₃ group, two CF₃ groups, or one CD₃ group and one CF₃ group.

In a particularly preferred embodiment, the compound of Formula 1 is perdeutero-Bisphenol A, ie. 2,2-Bis(4-hydroxyphenyl)propane-d₁₆.

In another particular embodiment there is provided the use of 2,2-Bis(4-hydroxyphenyl)propane-d₁₆, as a tracer in sealing compositions for Bisphenol A-containing surfaces.

In yet another embodiment, the compound of Formula 1 is 2,2-Bis(4-hydroxyphenyl)propane-*methyl*-d₆ (i.e. R₁, R₂, R₃ and R₄ are all hydrogen and G is a carbon atom linked to two CD₃ groups).

In **a second aspect** of the invention there is provided a coating composition comprising the following three components:
1. an epoxysiloxane of formula

   R₁-Si(OR₂)₃

   wherein
   R₁ = a C₅ to C₃₀ alkyl group containing one or more epoxy groups,
   R₂ = a C₁ to C₆ alkyl group, and
2. an aminosiloxane of formula

   R₃-Si(OR₄)₃

   wherein
   R₃ = a C₅ to C₃₀ alkyl group containing one or more amino groups,
   R₄ = a C₁ to C₆ alkyl group, and
3. a compound of Formula I according to the first aspect of the present invention.

In a particular embodiment of the second aspect of the invention, the coating composition contains two or more compounds of Formula I according to the first aspect of the present invention.

In a **third aspect** of the invention there is provided a process for treating surfaces comprising the steps of:
a. optionally applying one or more layers of a primer;
b. forming a first barrier over a surface, the first barrier comprising a first coating composition, a tracer substance according to the first aspect of the invention, and optionally having a first color;
c. once the first barrier has cured, forming a second barrier over the first barrier, the second barrier comprising a second coating composition, and optionally having a second color, and;
d. once the second barrier has cured, forming a third barrier over the second barrier, the third barrier comprising a third coating composition, and optionally having a third color.

A coating composition of the present invention should have a high density signifying a dense structrure that prevent fast diffusion of compounds through the layer. It should also have a low solubility for the SVC. Materials based on silicates, alumina silicates and/or other mixed metal oxide films containing mixtures of Si, Ti and Zr oxides will have these properties. To ensure a sufficient flexibility of the films, one or more organic compounds should be present in the film. Such compounds will preferentially contain an aromatic or heteroaromatic moiety with two or more reactive organometallic sites capable of linking into the metal oxide network. The curing process can be initiated by the prescence of moisture or by photoinitialisers.

The one or more colors optionally used in the process according to the third aspect of the present invention may be Fluorescent Dyes, such as UV-, IR- or near-IR Fluorescent Dyes.
In a particular embodiment of the third aspect of the invention there is provided a process for treating surfaces, comprising the steps of:
a. optionally applying one or more layers of a primer;
b. forming a first barrier over a surface, the first barrier comprising a first coating composition according to the second aspect of the present invention, and optionally having a first color;
c. once the first barrier has cured, forming a second barrier over the first barrier, the second barrier comprising a second coating composition, and optionally having a second color, and
d. once the second barrier has cured, forming a third barrier over the second barrier, the third barrier comprising a third coating composition, and optionally having a third color,
wherein the second and third barriers do not contain a tracer substance according to the present invention, and may be formed using any suitable curing coating composition compatible with the silicate-based coating composition according to the second aspect of the present invention.

Surfaces to be treated according to the third aspect of the invention comprise both porous and non-porous surfaces. Examples of porous surfaces comprise **concrete** (medium to high porosity), **brick** (very high porosity), and examples of non-porous surfaces comprise **wood** (low porosity) and **painted surfaces** (very low porosity). For porous surfaces it is recommended to apply one or more layers of a primer before the first layer of a coating composition.

In a **fourth aspect** there is provided a multi-layered barrier covering a surface comprising two or more cured or hardened layers of one or more coating compositions, wherein the first applied layer of coating composition contains a tracer substance according to the first aspect of the invention. A multi-layered barrier according to the fourth aspect will typically have a thickness of between 250-300 µm, including the first applied layer of coating composition containing a tracer substance.

In a preferred embodiment the multi-layered barrier according to the fourth aspect of the present invention comprises a cured layer of coating composition according to the second aspect of the invention.

As each layer of a multi-layered barrier according to the present invention may be prepared by a number of different application methods, eg by brush or spray, and as the nature and porosity of the surface to be coated will influence the exact thickness of each layer and the interaction beween the applied layers, it is not meaningful to describe the multi-layered barrier according to the present invention in terms of absolute measurements of coat thickness etc., but rather as a result of following the instructions according to the third aspect of the invention.

Therefore, in another embodiment the multi-layered barrier according to the fourth aspect of the present invention is obtainable by the process described in the third aspect of the invention. In a **fifth aspect** of the invention there is provided a coating composition kit, comprising the following parts:
a. Component 1 admixed with one or more tracer substances according to the first aspect of the invention,
b. Component 1 without tracer substance,
c. Component 2, and
d. A set of instructions for mixing Component 1 and 2, and for carrying out the surface treating process according to the second aspect of the invention

Component 1 (with and without added tracer substance) and Component 2 of the kit are separate parts, and may for example be held in separate storage containers such as bottles or canisters. The kit according to the fifth aspect may also contain the one or more tracer substances admixed with Component 2 instead of Component 1.

The kit according to the fifth aspect may also contain the tracer substance as a separate part, i.e. not admixed with either Component 1 or Component 2, for example as a solution in ethanol or another suitable solvent.

The kit according to the fifth aspect may contain as Component 1:
- an epoxysiloxane of formula

   R₁-Si(OR₂)₃

   wherein
   R₁ = a C₅ to C₃₀ alkyl group containing one or more epoxy groups,
   R₂ = a C₁ to C₆ alkyl group,
and as Component 2:
- an aminosiloxane of formula

   R₃-Si(OR₄)₃

   wherein
   R₃ = a C₅ to C₃₀ alkyl group containing one or more amino groups,
   R₄ = a C₁ to C₆ alkyl group,
and additionally:
- one or more tracer substances according to the first aspect of the invention, optionally as a solution in ethanol or another suitable solvent,
- A set of instructions:
   ∘ For applying the one or more tracer substances to the surface to be treated,
   ∘ for mixing Component 1 and 2, and
   ∘ for carrying out the surface treating process according to the second aspect of the invention

In a **sixth aspect** there is provided an analytical method for field testing surfaces covered by a multi-layered barrier according to the fourth aspect aspect of the present invention, in order to determine the co-presence of an SVC (such as a PCB) and a tracer substance according to the first aspect of the invention, using automated test equipment including a suitable gas chromatographic (GC) column connected to a mass spectrometric (MS) detector and a microprocessor including a memory in which is located data regarding magnitudes and retention times for a plurality of standard SVC mixtures and tracer substances, said method comprising the following steps:
a. preparing a sample by using a High Volume Sampler (HVS) for SVCs in ambient air, or a standardized wipe sample for surfaces potentially contaminated with SVCs;
b. separating and analyzing the components in said sample on a said gas chromatographic column using said MS detector which has an input for receiving said sample and an output for providing separated component peaks;
c. providing an electrical output signal including information regarding the magnitude and retention time of component peaks using said mass spectrometric detector which is connected to the output of said GC column;
d. comparing said electrical output with the contents of said memory through the use of said microprocessor; and
e. indicating one of the following:
   i. the presence of said SVC in said analytical sample based upon comparison with data in said microprocessor memory,
   ii. the co-presence of said SVC in said analytical sample together with said tracer substance or,
   iii. no SVC or tracer is present in the analytical sample,
   wherein the co-presence of an SVC together with the tracer substance (option e-ii) is indicative of cracks or other faults in said multi-layer barrier, whereas the presence of an SVC alone indicates a new source of the SVC.

### EXAMPLES

### Example 1: Tracer substances.

### Example 1a:

**4,4'-bis(2,2,2-trifluoroethoxy)-1,1'-biphenyl** was prepared analogously to 1,4-bis(2,2,2-trifluoroethoxy)-benzene (Reference: A K Ray et al. US 6458957 B1, Example 1), starting from 20 gr (65 mmol) 4,4'-dibromo-1,1'-biphenyl (CAS 92-86-4) instead of 1,4-dibromobenzene. Yield: quantitative, mp. 115°C (ethanol), bp. app. 367 °C
Analysis: ¹H NMR (400 MHz, CDCl₃, tetramethylsilane int.ref) 4.38 ppm 4H q (-OCH₂-CF₃), 7.0 ppm 4H d (aromatic protons in 3, 3', 5 and 5' position), 7.5 ppm 4H d (aromatic protons in 2, 2', 6 and 6' positions).
Mass spectrometry: m/z = 350, 267 (100%), 239, 156, 128.

### Example 1b:

**4,4'-dimethoxy-1,1'-biphenyl-*d₆*,** is prepared from 4,4'-dihydroxy-1,1'-biphenyl (CAS 92-88-6) by alkylation with methyl iodide-*d3*.

### Example 1c:

**2,2-Bis(4-hydroxyphenyl)propane-*methyl*-d₆** (Bisphenol A-(*dimethyl*-d₆))
The target compound can be synthesized by condensation of hexadeutero acetone and phenol under acidic conditions, and is also commercially available (CAS 86588-58-1).

### Example 1d:

**4,4'-(4,4,4-trifluorobutane-2,2-diyl)diphenol**
The target compound is synthesized by condensation of 4,4,4-trifluorobutan-2-one (CAS 2366-70-3) and phenol under acidic conditions.

### Example 1e:

**Bisphenol A-d₁₆** (2,2-Bis(4-hydroxyphenyl)propane-d₁₆)
Commercially available (CAS Number 96210-87-6)

### Example 2: Use of sealing compositions comprising tracer substances

The surface to be sealed should be dry and free of dust. Surfaces which are very smooth or slippery can advantageously be roughened lightly with sanding paper or the like before applying the coating composition, to ensure a good adherence.

A typical sealant according to the present invention is a two-component silicate based coating composition, comprising an epoxysiloxane component and an aminosiloxane component. The two components are mixed shortly before the application to the surface.

For the first layer of coating composition to be applied, a relevant tracer substance according to the present invention is either added to the final mixture, or is present in one of the two components before mixing. Alternatively, the tracer substance may be applied neat or as a solution in a suitable solvent like eg. ethanol or cyclohexane/acetone 1:1 before the first layer of coating composition is applied to the surface.

For porous surfaces it is recommended to apply one or more layers of a suitable primer before applying the first layer of the coating composition.

The application of the coating composition can be performed using a paint brush, paint roll or by spraying the coating composition onto the intended surface. It is important that fresh paint tools are employed after applying the first layer in order to prevent small amounts of tracer substance to be added to the second and subsequent layers of coating composition.
Typically at least two layers of coating composition are applied, and normally three layers. The first layer to be applied contains a suitable amount of tracer compound, typically between 25-1000 ppm. The second (and subsequent) layer contains no tracer compound.

The first layer of coating composition containing the tracer substance is allowed to cure for about an hour, or until the surface is only slightly sticky. The second layer of coating composition is then applied (containing no tracer), and after another curing period of about an hour, a third layer of coating composition may be applied. Additional layers may be applied.

The resulting multilayer barrier is allowed to fully cure (about 24 hrs at 12°C) before any further work is performed on the surface.

### Example 3: Test for integrity of the multilayer barrier

The first tests for the presence of SVCs are performed 1 month after the application of the sealant, as described above, to avoid re-volatization from secondary and tertiary surfaces.

Due to the low concentrations of SVCs in ambient air, large air volumes must be collected. Air volumes of about 1000-2000 m³ are frequently used, with typical flow rates of 25 m³/h. A High Volume Sampler (HVS), equipped with a glass-fibre filter for the collection of particulates and a polyurethane foam (PUF) adsorbent for sampling the semi-volatile compounds in the vapour phase, is the most suitable technique for the sampling of SVCs. The adsorbents commonly used for sampling SVCs in ambient air are PUF and an amberlite polymer XAD-2, which is more effective for trapping the more volatile SVCs.

As SVC partitioning between the gas phase and the particulate phase may be affected by the duration of the sampling, the total atmospheric concentration (i.e. the sum of the filter-retained and adsorbent-retained compounds) should be reported. A sampling time of 24 hours should give a rough estimate of the gas/particulate partitioning of the SVCs.

The recommended sampling time for air using an HVS is between 24-48 hours. When only a limited number of samples can be taken within a given time period (for example due to budgetary constraints) it is suggested that one 24h sample is taken every 5 to 7 days. This would limit the number of samples to be analysed each year to 50 - 70; thus, seasonal changes may be detectable and it should be possible to calculate a reasonably reliable annual average concentration. Alternatively, the monitoring strategy could be based on short intensive measuring campaigns; for example, two campaigns (summer and winter) could be used to obtain information about the main seasonal changes and also about variations in concentration under different meteorological conditions. When re-volatization is important three campaigns could be considered; for example, one in winter, one in late spring/early summer (May-June) when ambient temperatures increase and one in late summer (August-September) when sea water temperatures reach a maximum.
Sampling can also be conducted as swab tests. If the surface to be sampled is smooth and impervious (e.g., unpainted metal surfaces), a wipe sample can be collected to determine if the surface is contaminated with SVCs. A standard wipe test uses a 10 cm by 10 cm (or equivalent that equals 100 cm²) template to outline the sample area and a gauze pad or glass wool that has been saturated with hexane or another suitable solvent like ethanol to collect the sample. The solvent-saturated wipe is used to thoroughly swab the area inside the 100 cm² template. Care must be taken to assure proper use of the sampling template, as the sample results will be based on the 100 cm² sample area (i.e., µg per 100 cm²).

SVC analysis is then performed by means of gas chromatography coupled with mass spectrometry (GC/MS) using automated test equipment including a gas chromatographic (GC) column connected to a mass spectrometric (MS) detector and a microprocessor including a memory in which is located data regarding magnitudes and retention times for a plurality of standard SVC mixtures and tracer substances.

The SVC concentrations in the samples should be adjusted for any losses which may have occurred during analysis, by using internal standards. Field blanks and quality control samples should also be used. The analysis shall include the specific tracer substance as an internal standard.

### Example 4: Test of tracer compounds under simulated compromised barrier conditions

A number of experiements were carried out with the purpose of 1) demonstrating that a coating composition according to Example 2 of the present invention can withhold both semi-volatile compounds (SVCs) and also compounds which are practically non-volatile, and 2) that tracer compounds according to the present invention behave sufficiently similar to the contaminants in question, such that their presence in an analysis indicate a compromised barrier.

The conclusion of the various tests was, that a coating composition according to Example 2 of the present invention can withhold both types of contaminants, in this case PCBs and Bisphenol A. The tests also demonstrated that it was possible to find tracer compounds with relevant properties for both contaminants.

As an example of SVCs, **PCBs** were tested in evaporation tests using a couple of commercial mixtures of PCB congeners and a standardized air sampling procedure. As an example of a non-volatile contaminant **Bisphenol A** was tested, using a swab test procedure.

The following two compounds were tested for their capacity as tracer substances for the PCBs and Bisphenol A (as mentioned above, the term "PCBs" cover any of 209 configurations (congeners) of a biphenyl ring with various amounts of chlorine atoms substituted around each ring (2 to 10 possible Cl atoms):
- 4,4'-bis(2,2,2-trifluoroethoxy)-1,1'-biphenyl
- 2,2-Bis(4-hydroxyphenyl)propane-*d₁₆* ("d-Bisphenol A")

The boiling point of 4,4'-bis(2,2,2-trifluoroethoxy)-1,1'-biphenyl is app 367 °C at ambient pressure. d-Bisphenol A has a boiling point of app. 220 °C/4 mmHg (for all practical purposes, Bisphenol A and its deuterated analogs are not volatile under the test conditions, and are only tested by a swab procedure).

### Reagents used

### PCB reference mixtures:

Dutch Seven PCB Mixture (NEN 5734/VPR C85-16, LGC Standards),
PCB mix (certified reference material bcr - 365, LGC Standards),
PCB202 2,2',3,3',5,5',6,6'-Octachlorobiphenyl (13C12,99%, LGC Standards)
Aroclor 1248 (certified reference material, 1000 µg/mL in isooctane, Sigma Aldrich)
DBOB 4,4'-Dibromooctafluorobiphenyl (Sigma Aldrich),

### Other reference compounds:

Bisphenol A (Sigma Aldrich)

### Tracer compounds:

4,4'-2,2,2-trifluoroethoxy biphenyl prepared according to Example 1a
Bisphenol A-*d₁₆* (Sigma Aldrich)

### Example 4.1 - PCB analysis by air sampling

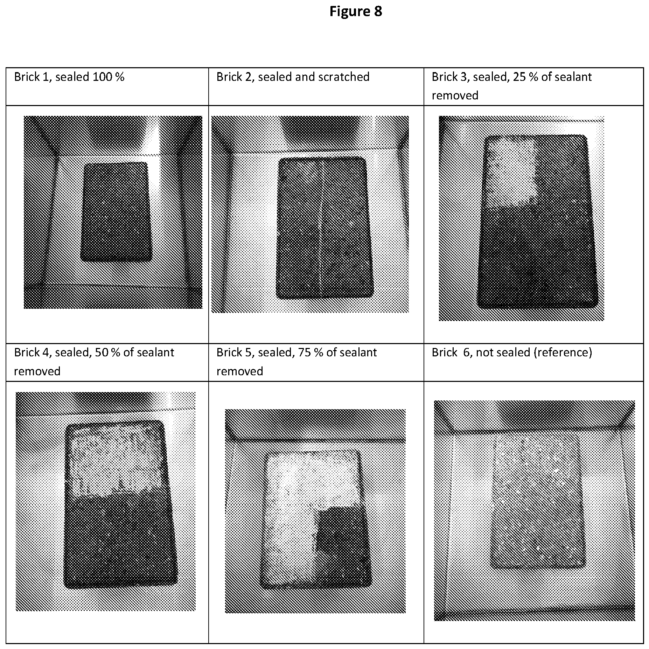

The experiment comprises 6 concrete bricks (see figure 8). Each brick had one surface treated with 1,1mL Aroclor 1000 ppm solution and 2mL of a 1000 ppm solution of the tracer substance 4,4'-2,2,2-trifluoroethoxy biphenyl. Subsequently 5 of the bricks were sealed twice with a coating composition according to Example 2, with an interval of 30 minutes between each coat. The sealing was finally allowed to cure for 24 hours. The bricks were then treated according to the legends in fig.8.

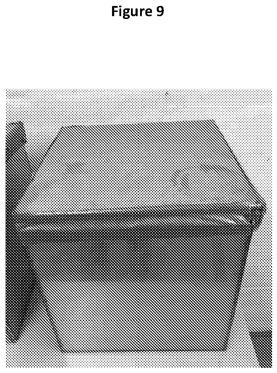

Subsequently each brick was placed in a stainless steelbox 30x30x30cm, see fig 9 (cleaned with acetone before use). The boxes were closed with duct tape and left standing for 30 minutes. An air sample was then taken from each box using a sampling tube with PUF/XAD-2/PUF-Sandwich sorbent material, with a flow of 2L/min for 2 hours. Finally each sampling tube was analysed using the method described in the analytical method section.

### The following individual PCB congeners and their isomers were analysed for:

| Representative compound | Congener # | CAS# | Boiling point (°C) |
|---|---|---|---|
| 2,4,4'-Trichlorobiphenyl | 28 | 7012-37-5 | 337 |
| 2,2',5,5'-Tetrachlorobiphenyl | 52 | 35693-99-3 | 360 |
| 2,2',4,5,5'-Pentachlorobiphenyl | 101 | 37680-73-2 | 381 |
| 2,3',4,4',5-Pentachlorobiphenyl | 118 | 31508-00-6 | 381 |
| 2,2',3,4,4',5'-Hexachlorbiphenyl | 138 | 35065-28-2 | 400 |
| 2,2',4,4',5,5'-Hexachlorobiphenyl | 153 | 35065-27-1 | 400 |
| 2,2',3,4,4',5,5'-Heptachlorobiphenyl | 180 | 35065-29-3 | 417 |

### Analytical method for PCB testing (by air sampling)

The sampling tube was transferred to a glass beaker, and 5 ml cyclohexane/acetone (1:1) was added together with an extraction standard 4,4'-Dibromooctafluorobiphenyl (DBOB). The beaker was then sonicated for 2 hours. 900 µL extract was mixed with 100 µL injection standard PCB 202 C¹³ and analysed by GC-MS in SIM mode. The concentration of the individual PCB congeners was determined based on peak area. The congener concentrations were finally added up and multiplied by 5 (a calibration factor). The concentration of the tracer substance 4,4'-2,2,2-trifluoroethoxy biphenyl was determined directly by peak area.

### Results of PCB analysis by air sampling:

| **Summarized results-PCB and tracer substance* in air** | | | |
|---|---|---|---|
| **Lab #** | **Sample** | **PCB conc in ng/m³** | **Tracer conc in ng/m³** |
| 1 | Brick 1, sealed 100 % | **640** | **N.D.** |
| 2 | Brick 2, sealed and scratched | **710** | **N.D.** |
| 3 | Brick 3, sealed, 25 % of sealant removed | **720** | **13** |
| 4 | Brick 4, sealed, 50 % of sealant removed | **775** | **17** |
| 5 | Brick 5, sealed, 75 % of sealant removed | **3910** | **118** |
| 6 | Brick 6, not sealed (reference) | **8950** | **219** |
| * 4,4'-2,2,2-trifluoroethoxy biphenyl, N.D. not detected | | | |

| **Detailed PCB results** | | | | | | | | | | |
|---|---|---|---|---|---|---|---|---|---|---|
| | | PCB congeners in ng/m3 | | | | | | | | |
| **Lab #** | **28** | **52** | **101** | **118** | **138** | **153** | **180** | Σ7 PCB | Factor | Total PCB |
| 1 | 90,0 | 38,0 | N.D. | N.D. | N.D. | N.D. | N.D. | 128,0 | 5,0 | **640** |
| 2 | 101,0 | 41,0 | N.D. | N.D. | N.D. | N.D. | N.D. | 142,0 | 5,0 | **710** |
| 3 | 106,0 | 38,3 | N.D. | N.D. | N.D. | N.D. | N.D. | 144,0 | 5,0 | **720** |
| 4 | 113.0 | 41.7 | N. D. | N. D. | N. D. | N. D. | N. D. | 155.0 | 5.0 | **775** |
| 5 | 383,0 | 333,0 | 65,3 | N.D. | N.D. | N.D. | N.D. | 781,0 | 5,0 | **3910** |
| 6 | 775,0 | 638,0 | 138,0 | 178,0 | 33,2 | 26,3 | N.D. | 1790,0 | 5,0 | **8950** |

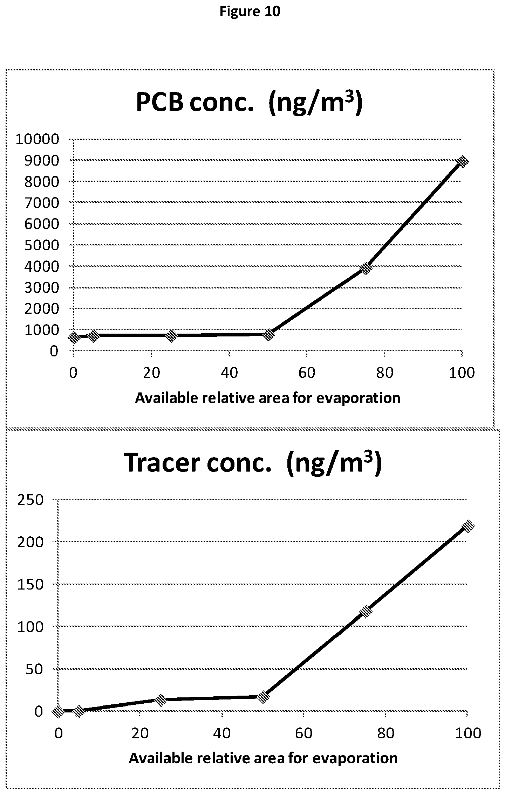

The above data have been plotted in the graphs shown in fig.10 and demonstrate the relation between the available relative area for evaporation and the measured air concentration of PCBs and the employed tracer substance 4,4'-2,2,2-trifluoroethoxy biphenyl. As can be seen from fig 10, the graphs for the tracer substance and PCBs have almost identical slope from the point where the available area for evaporation reaches about 50%. This indicates that 4,4'-2,2,2-trifluoroethoxy biphenyl is a suitable tracer substance for PCBs.

### Example 4.2 - PCB analysis by swab testing

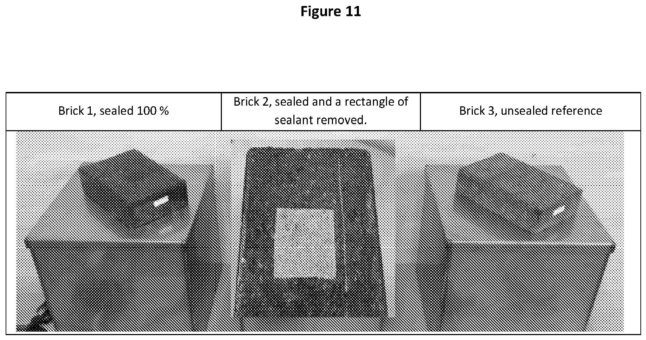

The experiment comprises 3 concrete bricks (see fig.11). Each brick had one surface treated with a 250 ppm solution of 4,4'-2,2,2-trifluoroethoxy biphenyl in 50/50 cyclohexane/acetone and with a 10.000 ppm solution of PCB. Subsequently 2 of the bricks were sealed twice with a coating composition according to Example 2, with an interval of 30 minutes between each coat. The last stone was used as an unsealed reference.

The sealing was allowed to cure for 24 hours, and the bricks were then treated according to the below table. Finally each brick was wiped with a cloth wetted with ethanol, and the cloth analysed using the method described in the analytical method section.

### Analytical method for swab testing of solid surfaces

The solid surface to be tested was wiped with a clean cloth wetted with ethanol, and the cloth transferred to a glass beaker. 40 ml cyclohexane/acetone (1:1) was added together with the extraction standard 4,4'-Dibromooctafluorobiphenyl (DBOB). The beaker was then sonicated for 2 hours. 900 µL extract was mixed with 100 µL injection standard PCB 202 C¹³ and the sample was analysed by GC-MS in SIM mode. The concentration of the individual components in the mass spectrum was determined based on peak area.

### Results of PCB analysis by swab testing:

| **Summarized results - PCB and tracer substance*** | | | | |
|---|---|---|---|---|
| **Lab #** | **Sample** | **PCB conc. in µg/m²** | **Tracer* conc. in µp/m²** | **Wiped area** |
| 1 | Brick 1 | **N.D.** | **N.D.** | 14X21cm |
| 2 | Brick 2 | **1070** | **8,9** | 14X21cm, sealant removed from an area of 5,5X5,7cm |
| 3 | Brick 3 | **13700** | **159** | 14X21cm |
| | | | | |
| * 4,4'-2,2,2-trifluoroethoxy biphenyl, N.D. not detected | | | | |

| **Detailed PCB results** | | | | | | | | | | |
|---|---|---|---|---|---|---|---|---|---|---|
| | PCB congener (µg/m²) | | | | | | | | | |
| **Lab #** | **28** | **52** | **101** | **118** | **138** | **153** | **180** | Σ7PCB | Factor | Total PCB |
| 1 | N. D. | N. D. | N. D. | N. D. | N. D. | N. D. | N. D. | - | 5,0 | **N.D.** |
| 2 | 160,0 | 44,7 | 3,910 | 3,990 | N. D. | N. D. | N. D. | 213,0 | 5,0 | **1070** |
| 3 | 2010 | 585,0 | 58,9 | 77,20 | N.D. | N.D. | N.D. | 2730 | 5,0 | **13700** |

The results of testing Brick 1 indicate that the sealing composition according to the present invention can withhold both PCBs and the tracer substance, 4,4'-2,2,2-trifluoroethoxy biphenyl as the analytical sample did not register any of these two compounds (Table row Lab#1). The results of testing Brick 2 indicate that applying even a very low amount of tracer substance (250 ppm vs. 10.000 ppm PCBs) is sufficient for a positive identification hereof together with identifying PCB congeners. 4,4'-2,2,2-trifluoroethoxy biphenyl can therefore for a range of PCB congeners be used as a tracer substance, the presence of which in an air sample or swab test may indicate a compromised barrier if used in accordance with Example 2.

### Example 4.3 - Bisphenol A analysis by swab testing

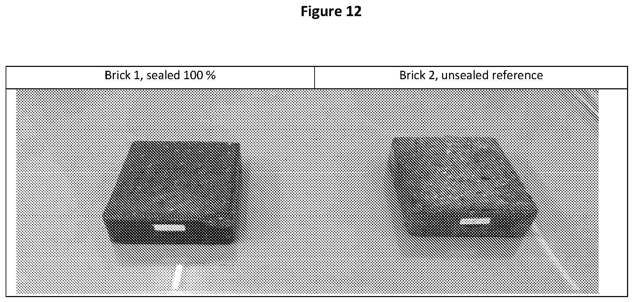

The experiment comprises 2 concrete bricks (see fig.12). Each brick had one surface treated with a 250 ppm solution of 4,4'-2,2,2-trifluoroethoxy biphenyl in 50/50 cyclohexane/acetone, a 1500 ppm solution of Bisphenol-A and a 1000 ppm solution of d-Bisphenol-A. Subsequently one of the bricks was sealed twice with a coating composition according to Example 2, with an interval of 30 minutes between each coat. The sealing was allowed to cure for 3 days. Each brick was then wiped with a cloth wetted with ethanol, and the cloth was analysed using the method described in the analytical method section.

### Analytical method for swab testing of solid surfaces

The same procedure was used as described in Example 4.2

### Results of Bisphenol A analysis by swab testing:

| **Results** - **Bisphenol-A, Bisphenol-A-d and tracer* by swab test** | | | | |
|---|---|---|---|---|
| **Sample** | **Bisphenol-A conc. µg/m²** | **Bisphenol-A-d conc µg/m²** | **Tracer* substance conc. µg/m²** | **Wiped area** |
| Brick 1 | N.D. | N.D. | N.D. | 14 x 21 cm |
| Brick 2 | **6000** | **5339** | **29** | 14 x 21 cm |
| * 4,4'-2,2,2-trifluoroethoxy biphenyl, N.D. not detected | | | | |

The results indicate that deuterated Bisphenol A can be used as a tracer substance for Bisphenol A, but also that the structurally unrelated compound 4,4'-2,2,2-trifluoroethoxy biphenyl can be used. It appears at a lower concentration in the analysis, but was also applied in a much lower concentration from the outset. As 4,4'-2,2,2-trifluoroethoxy biphenyl can also be used as a tracer substance for more volatile contaminants (see above example 4.1), this substance may be used as a generic tracer, the presence of which in an air sample or swab test may indicate a compromised barrier if used in accordance with Example 2. The results of testing Brick 1 further indicate that the sealing composition according to the present invention can withhold both Bisphenol A and the tracer substance, 4,4'-2,2,2-trifluoroethoxy biphenyl.

### Summary of Example 4:

The tests performed under various simulated compromised barrier conditions with the tracer substances 4,4'-2,2,2-trifluoroethoxy biphenyl and Bisphenol A-*d₁₆* indicate that these tracers behave very similar to the relevant SVCs, PCB and Bisphenol A, and that the tracer 4,4'-2,2,2-trifluoroethoxy biphenyl can be used for both air sampling and swab testing of solid surfaces.

## Claims

1. Use of a compound of Formula I as a tracer substance in coating compositions: 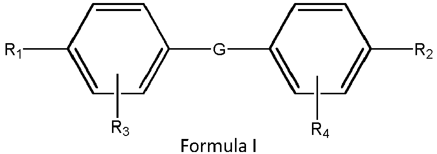 wherein
R₁ is selected from hydrogen, deuterium, hydroxy, C₁-C₆ alkoxy, trifluoromethoxy, 1,1,1-trifluoroethoxy or 1,1,1-trifluoropropoxy, and
R₂ is selected from hydrogen, deuterium, hydroxy, C₁-C₆ alkoxy, trifluoromethoxy, 1,1,1-trifluoroethoxy or 1,1,1-trifluoropropoxy, and
R₃ is selected from hydrogen, deuterium, fluorine, C₁-C₆ alkyl, trifluoromethyl or trifluoromethoxy, and
R₄ is selected from hydrogen, deuterium, fluorine, C₁-C₆ alkyl, trifluoromethyl or trifluoromethoxy,
and wherein
the linker group G may be absent, i.e. the two aromatic rings are directly linked by a single bond, or
the linker group G is a carbon atom linked to 1 or 2 C₁-C₆ alkyl groups optionally substituted with up to 6 fluorine atoms and/or up to 6 deuterium atoms,
and wherein the compound contains at least one deuterium atom and/or one fluorine atom.

2. A compound as defined in Claim 1 which is 4,4'-bis(2,2,2-trifluoroethoxy)-1,1'-biphenyl, i.e. a compound having the following structure:

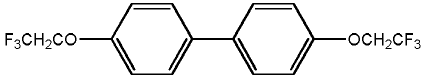

3. Use according to claim 1 of 2,2-Bis(4-hydroxyphenyl)propane-*d₁₆* as a tracer substance in coating compositions, i.e. a compound having the following structure:

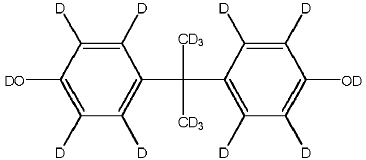

4. Use according to claim 1 of 4,4'-bis(2,2,2-trifluoroethoxy)-1,1'-biphenyl, as a tracer substance in coating compositions, i.e. a compound having the following structure:

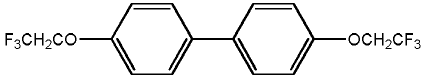

5. A coating composition comprising:
1. an epoxysiloxane of formula
R₁-Si(OR₂)₃
wherein
R₁ = a C₅ to C₃₀ alkyl group containing one or more epoxy groups,
R₂ = a C₁ to C₆ alkyl group, and
2. an aminosiloxane of formula
R₃-Si(OR₄)₃
wherein
R₃ = a C₅ to C₃₀ alkyl group containing one or more amino groups,
R₄ = a C₁ to C₆ alkyl group, and
3. a compound of Formula I as defined in any one of claim 1-3,
wherein the compound of Formula I is used as a tracer substance.

6. A process for treating surfaces comprising the steps of:
a. optionally applying one or more layers of a primer;
b. forming a first barrier over a surface, the first barrier comprising a first coating composition and a compound of Formula I as defined in any one of claim 1-3, and
c. once the first barrier has cured, forming a second barrier over the first barrier, the second barrier comprising a second coating composition, and
d. once the second barrier has cured, forming a third barrier over the second barrier, the third barrier comprising a third coating composition,
wherein the compound of Formula I is used as a tracer substance.

7. A process for treating surfaces according to claim 6, wherein the first coating composition is a coating composition according to claim 5.

8. A multi-layered barrier covering a surface comprising two or more cured or hardened layers of one or more coating compositions, wherein the coating composition employed in the first applied layer is prepared by a process involving the use of a compound of Formula I as a tracer substance.

9. A multi-layered barrier according to claim 8, wherein the first applied layer of coating composition is a coating composition according to claim 5.

10. A multi-layered barrier according to any one of claim 8-9 comprising at least three cured or hardened layers of one or more coating compositions.

11. A multi-layered barrier according to any one of claim 8-10 obtainable by the process of any one of claim 6-7.

12. A coating composition kit, comprising the following parts:
• Component 1 admixed with a compound of Formula I according to any one of claim 1-3
• Component 1 alone,
• Component 2, and
• A set of instructions for mixing Component 1 and 2, and for carrying out the surface treating process according to any one of claim 6-7, and
wherein Component 1 (with and without added compound of Formula I) and Component 2 of the kit are separate parts, and may be held in separate storage containers such as bottles or canisters, and wherein the compound of Formula I is used as a tracer substance.

13. An analytical method for field testing surfaces covered by a multi-layered barrier according to any one of claim 8-11 to determine the co-presence of an SVC and one or more tracer substances of Formula I as defined in any one of claims 1-3, using automated test equipment including a gas chromatographic (GC) column connected to a mass spectrometric (MS) detector and a microprocessor including a memory in which is located data regarding magnitudes and retention times for a plurality of standard SVC mixtures and tracer substances, said method comprising the following steps:
a. preparing a sample by using a High Volume Sampler (HVS) for SVCs in ambient air, or a standardized wipe sample for surfaces potentially contaminated with SVCs;
b. separating and analyzing the components in said sample on a said gas chromatographic column using said MS detector which has an input for receiving said sample and an output for providing separated component peaks;
c. providing an electrical output signal including information regarding the magnitude and retention time of component peaks using said mass spectrometric detector which is connected to the output of said GC column;
d. comparing said electrical output with the contents of said memory through the use of said microprocessor; and
e. indicating one of the following:
i. the presence of said SVC in said analytical sample based upon comparison with data in said microprocessor memory,
ii. the co-presence of said SVC in said analytical sample together with said one or more tracer substances or,
iii. no SVC or tracer substance is present in the analytical sample,
wherein the co-presence in the analytical sample of said SVC together with said one or more tracer substances is indicative of cracks or other faults in said multi-layer barrier, whereas the presence of said SVC alone in the analytical sample indicates a new source of the SVC.

## Patentansprüche

1. Verwendung einer Verbindung der Formel I als Indikatorsubstanz in Beschichtungszusammensetzungen: 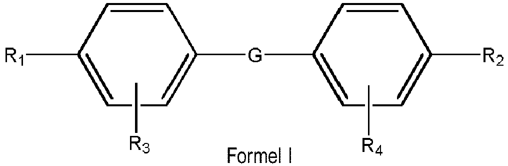 wobei
R₁ aus Wasserstoff, Deuterium, Hydroxy, C₁-C₆-Alkoxy, Trifluormethoxy, 1,1,1-Trifluorethoxy oder 1,1,1-Trifluorpropoxy ausgewählt ist und
R₂ aus Wasserstoff, Deuterium, Hydroxy, C₁-C₆-Alkoxy, Trifluormethoxy, 1,1,1-Trifluorethoxy oder 1,1,1-Trifluorpropoxy ausgewählt ist und
R₃ aus Wasserstoff, Deuterium, Fluor, C₁-C₆-Alkyl, Trifluormethyl oder Trifluormethoxy ausgewählt ist und R₄ aus Wasserstoff, Deuterium, Fluor, C₁-C₆-Alkyl, Trifluormethyl oder Trifluormethoxy ausgewählt ist und wobei
die Verbindungsgruppe G fehlen kann, d. h. die zwei aromatischen Ringe durch eine Einfachbindung direkt verbunden sind, oder
die Verbindungsgruppe G für ein Kohlenstoffatom steht, das mit 1 oder 2 C₁-C₆-Alkylgruppen verbunden ist, die gegebenenfalls durch bis zu 6 Fluoratome und/oder bis zu 6 Deuteriumatome substituiert sind,
und wobei die Verbindung mindestens ein Deuteriumatom und/oder ein Fluoratom enthält.

2. Verbindung gemäß der Definition in Anspruch 1, bei der es sich um 4,4'-Bis(2,2,2-trifluorethoxy)-1,1'-biphenyl handelt, d. h. eine Verbindung mit der folgenden Struktur:

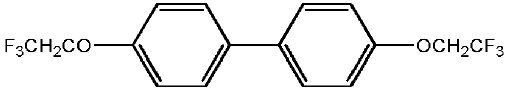

3. Verwendung nach Anspruch 1 von 2,2-Bis(4-hydroxyphenyl)propan-*d₁₆* als Indikatorsubstanz in Beschichtungszusammensetzungen, d. h. eine Verbindung mit der folgenden Struktur:

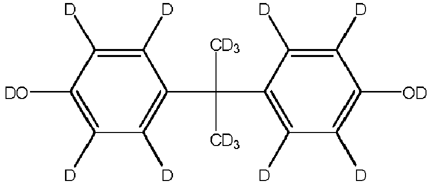

4. Verwendung nach Anspruch 1 von 4,4'-Bis(2,2,2-trifluorethoxy)-1,1'-biphenyl als Indikatorsubstanz in Beschichtungszusammensetzungen, d. h. eine Verbindung mit der folgenden Struktur:

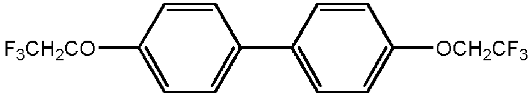

5. Beschichtungszusammensetzung, umfassend:
1. ein Epoxysiloxan der Formel
R₁-Si (OR₂)₃
wobei
R₁ = eine C₅- bis C₃₀-Alkylgruppe, die eine oder mehrere Epoxygruppen enthält,
R₂ = eine C₁- bis C₆-Alkylgruppe, und
2. ein Aminosiloxan der Formel
R₃-Si (OR₄)₃
wobei
R₃ = eine C₅- bis C₃₀-Alkylgruppe, die eine oder mehrere Aminogruppen enthält,
R₄ = eine C₁- bis C₆-Alkylgruppe, und
3. eine Verbindung der Formel I gemäß der Definition in einem der Ansprüche 1-3,
wobei die Verbindung der Formel I als Indikatorsubstanz verwendet wird.

6. Verfahren zum Behandeln von Oberflächen, umfassend die folgenden Schritte:
a. gegebenenfalls Aufbringen einer oder mehrerer Schichten einer Grundierung;
b. Bilden einer ersten Barriere über einer Oberfläche, wobei die erste Barriere eine erste Beschichtungszusammensetzung und eine Verbindung der Formel I gemäß der Definition in einem der Ansprüche 1-3 umfasst, und
c. sobald die erste Barriere ausgehärtet ist, Bilden einer zweiten Barriere über der ersten Barriere, wobei die zweite Barriere eine zweite Beschichtungszusammensetzung umfasst, und
d. sobald die zweite Barriere ausgehärtet ist, Bilden einer dritten Barriere über der zweiten Barriere, wobei die dritte Barriere eine dritte Beschichtungszusammensetzung umfasst,
wobei die Verbindung der Formel I als Indikatorsubstanz verwendet wird.

7. Verfahren zum Behandeln von Oberflächen nach Anspruch 6, wobei die erste Beschichtungszusammensetzung eine Beschichtungszusammensetzung nach Anspruch 5 ist.

8. Mehrschichtige Barriere, die eine Oberfläche bedeckt, umfassend zwei oder mehr ausgehärtete oder gehärtete Schichten einer oder mehrerer Beschichtungszusammensetzungen, wobei die Beschichtungszusammensetzung, die in der ersten aufgebrachten Schicht eingesetzt wird, durch ein Verfahren hergestellt wird, das die Verwendung einer Verbindung der Formel I als Indikatorsubstanz beinhaltet.

9. Mehrschichtige Barriere nach Anspruch 8, wobei es sich bei der ersten aufgebrachten Schicht von Beschichtungszusammensetzung um eine Beschichtungszusammensetzung nach Anspruch 5 handelt.

10. Mehrschichtige Barriere nach einem der Ansprüche 8-9, umfassend mindestens drei ausgehärtete oder gehärtete Schichten einer oder mehrerer Beschichtungszusammensetzungen.

11. Mehrschichtige Barriere nach einem der Ansprüche 8-10, erhältlich durch das Verfahren nach einem der Ansprüche 6-7.

12. Beschichtungszusammensetzungskit, umfassend die folgenden Teile:
• Komponente 1, vermischt mit einer Verbindung der Formel I nach einem der Ansprüche 1-3,
• ausschließlich Komponente 1,
• Komponente 2 und
• eine Gruppe von Anweisungen zum Mischen von Komponente 1 und 2 und zum Durchführen des Oberflächenbehandlungsverfahrens nach einem der Ansprüche 6-7, und
wobei Komponente 1 (mit und ohne zugegebene Verbindung der Formel I) und Komponente 2 des Kits separate Teile sind und in separaten Lagerbehältern wie etwa Flaschen oder Kanistern aufbewahrt werden können und wobei die Verbindung der Formel I als Indikatorsubstanz verwendet wird.

13. Analyseverfahren für Feldversuche an Oberflächen, die durch eine mehrschichtige Barriere nach einem der Ansprüche 8-11 bedeckt sind, um das gleichzeitige Vorhandensein einer SVC (Semi-Volatile Compound - halbflüchtige Verbindung) und einer oder mehrerer Indikatorsubstanzen der Formel I gemäß der Definition in einem der Ansprüche 1-3 zu bestimmen, unter Verwendung von automatisierter Versuchsausrüstung, die eine Gaschromatographie(GC)-Säule beinhaltet, die mit einem Massenspektrometrometrie(MS)-Detektor und einem Mikroprozessor verbunden ist, der einen Speicher beinhaltet, in dem sich Daten bezüglich Größenordnungen und Retentionszeiten für eine Vielzahl von Standard-SVC-Gemischen und Indikatorsubstanzen befinden, wobei das Verfahren die folgenden Schritte umfasst:
a. Herstellen von einer Probe unter Verwendung eines High-Volume-Samplers (HVS) für SVCs in Umgebungsluft oder von einer standardisierten Wischprobe für Oberflächen, die potenziell mit SVCs verunreinigt sind;
b. Trennen und Analysieren der Komponenten in der Probe an einer Gaschromatographiesäule unter Verwendung des MS-Detektors, der eine Eingabe zum Aufnehmen der Probe und eine Ausgabe zum Bereitstellen getrennter Komponentenpeaks aufweist;
c. Bereitstellen eines elektrischen Ausgabesignals, das Informationen bezüglich der Größenordnung und Retentionszeit von Komponentenpeaks unter Verwendung des Massenspektrometriedetektors beinhaltet, der mit der Ausgabe der GC-Säule verbunden ist;
d. Vergleichen der elektrischen Ausgabe mit dem Inhalt des Speichers durch die Verwendung des Mikroprozessors; und
e. Anzeigen eines der Folgenden:
i. Vorhandensein der SVC in der Analyseprobe basierend auf dem Vergleich mit Daten in dem Mikroprozessorspeicher,
ii. gleichzeitiges Vorhandensein der SVC in der Analyseprobe zusammen mit der einen oder den mehreren Indikatorsubstanzen oder
iii. in der Analyseprobe ist keine SVC oder Indikatorsubstanz vorhanden,
wobei das gleichzeitige Vorhandensein der SVC zusammen mit der einen oder den mehreren Indikatorsubstanzen in der Analyseprobe auf Risse oder andere Fehler in der mehrschichtigen Barriere hindeutet, während das Vorhandensein ausschließlich von SVC in der Analyseprobe auf eine neue Quelle der SVC hindeutet.

## Revendications

1. Utilisation d'un composé selon la Formule I comme substance de marquage dans des compositions de revêtement : 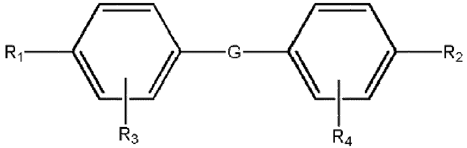 Formule I
dans laquelle
R₁ est sélectionné parmi un atome d'hydrogène ou de deutérium ou un groupe hydroxy, alcoxy C₁-C₆, trifluorométhoxy, 1,1,1-trifluoroéthoxy, ou 1,1,1-trifluoropropoxy, et
R₂ est sélectionné parmi un atome d'hydrogène ou de deutérium ou un groupe hydroxy, alcoxy C₁-C₆, trifluorométhoxy, 1,1,1-trifluoroéthoxy, ou 1,1,1-trifluoropropoxy, et
R₃ est sélectionné parmi un atome d'hydrogène, de deutérium ou de fluor ou un groupe alkyle C₁-C₆, trifluorométhyle ou trifluorométhoxy, et
R₄ est sélectionné parmi un atome d'hydrogène, de deutérium ou de fluor ou un groupe alkyle C₁-C₆, trifluorométhyle ou trifluorométhoxy,
et dans laquelle
le groupe de liaison G peut être absent, c'est-à-dire que les deux cycles aromatiques sont liés directement par une liaison simple, ou
le groupe de liaison G est un atome de carbone lié à 1 ou 2 groupes alkyle C₁-C₆ optionnellement substitués par au maximum 6 atomes de fluor et/ou au maximum 6 atomes de deutérium,
et dans laquelle le composé contient au moins un atome de deutérium et/ou un atome de fluor.

2. Composé tel que défini dans la revendication 1, lequel est le 4,4'-bis(2,2,2-trifluoroéthoxy)-1,1'-biphényle, c'est-à-dire un composé ayant la structure suivante :

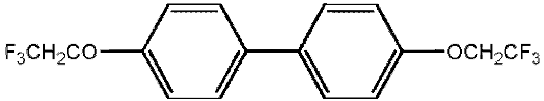

3. Utilisation selon la revendication 1 de 2,2-bis(4-hydroxyphényl)propane-*d₁₆* comme substance de marquage dans des compositions de revêtement, c'est-à-dire d'un composé ayant la structure suivante :

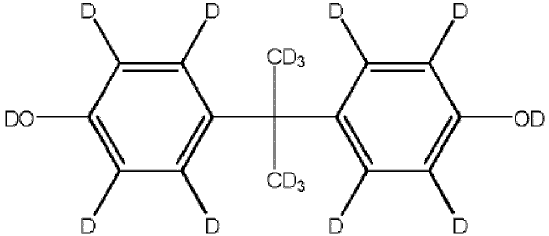

4. Utilisation selon la revendication 1 de 4,4'-bis(2,2,2-trifluoroéthoxy)-1,1'-biphényle comme substance de marquage dans des compositions de revêtement, c'est-à-dire d'un composé ayant la structure suivante :

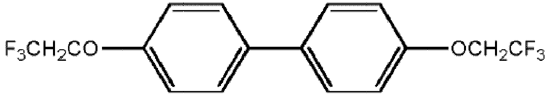

5. Composition de revêtement comprenant :
1. un époxysiloxane selon la Formule
R₁-Si(OR₂)₃
dans laquelle
R₁ est un groupe alkyle C₅-C₃₀ contenant un ou plusieurs groupes époxy,
R₂ est un groupe alkyle C₁-C₆, et
2. un aminosiloxane selon la Formule
R₃-Si(OR₄)₃
dans laquelle
R₃ est un groupe alkyle C₅-C₃₀ contenant un ou plusieurs groupes amino,
R₄ est un groupe alkyle C₁-C₆, et
3. un composé selon la Formule I tel que défini dans l'une quelconque des revendications 1 à 3, dans laquelle le composé selon la Formule I est utilisé comme substance de marquage.

6. Procédé de traitement de surfaces, comprenant les étapes qui consistent à :
a. optionnellement, appliquer une ou plusieurs couches d'un apprêt ;
b. former une première barrière sur une surface, la première barrière comprenant une première composition de revêtement et un composé selon la Formule I tel que défini dans l'une quelconque des revendications 1 à 3, et
c. une fois que la première barrière est polymérisée, former une deuxième barrière par-dessus la première barrière, la deuxième barrière comprenant une deuxième composition de revêtement, et
d. une fois que la deuxième barrière est polymérisée, former une troisième barrière par-dessus la deuxième barrière, la troisième barrière comprenant une troisième composition de revêtement,
dans laquelle le composé selon la Formule I est utilisé comme substance de marquage.

7. Procédé de traitement de surfaces selon la revendication 6, dans lequel la première composition de revêtement est une composition de revêtement selon la revendication 5.

8. Barrière multicouche recouvrant une surface comprenant deux couches ou plus polymérisées ou durcies d'une ou plusieurs compositions de revêtement, dans laquelle la composition de revêtement utilisée dans la première couche appliquée est préparée par un procédé impliquant l'utilisation d'un composé selon la Formule I comme substance de marquage.

9. Barrière multicouche selon la revendication 8, dans laquelle la première couche appliquée de composition de revêtement est une composition de revêtement selon la revendication 5.

10. Barrière multicouche selon l'une quelconque des revendications 8 ou 9, comprenant au moins trois couches polymérisées ou durcies d'une ou plusieurs compositions de revêtement.

11. Barrière multicouche selon l'une quelconque des revendications 8 à 10, pouvant être obtenue par le procédé selon l'une quelconque des revendications 6 ou 7.

12. Kit de composition de revêtement, comprenant les éléments suivants :
• Constituant 1 mélangé par addition avec un composé selon la Formule I selon l'une quelconque des revendications 1 à 3,
• Constituant 1 seul,
• Constituant 2, et
• un ensemble d'instructions pour mélanger le Constituant 1 et le Constituant 2, et pour exécuter le procédé de traitement de surfaces selon l'une quelconque des revendications 6 ou 7, et dans lequel le Constituant 1 (avec et sans composé ajouté selon la Formule I) et le Constituant 2 du kit sont des éléments distincts, et peuvent être conservés dans des contenants de stockage distincts tels que des flacons ou des cartouches, et dans lequel le composé selon la Formule I est utilisé comme substance de marquage.

13. Méthode d'analyse pour tester sur le terrain des surfaces recouvertes d'une barrière multicouche selon l'une quelconque des revendications 8 à 11, afin de déterminer la co-présence d'un CSV (contaminant semi-volatil) et d'une ou plusieurs substances de marquage selon la Formule I telles que définies dans l'une quelconque des revendications 1 à 3, en utilisant un équipement de test automatisé comprenant une colonne de chromatographie gazeuse (CG) connectée à un détecteur par spectrométrie de masse (SM) et à un microprocesseur contenant une mémoire dans laquelle se trouvent des données concernant des grandeurs et des temps de rétention pour une pluralité de mélanges standard de CSV et de substances de marquage, ladite méthode comprenant les étapes suivantes :
a. la préparation d'un échantillon en utilisant un échantillonneur à grand débit pour les CSV dans l'air ambiant, ou un échantillon normalisé prélevé par essuyage pour les surfaces potentiellement contaminées par des CSV ;
b. la séparation et l'analyse des constituants dans ledit échantillon sur ladite colonne de chromatographie gazeuse en utilisant ledit détecteur par SM comportant une entrée pour recevoir ledit échantillon et une sortie pour produire des pics pour les constituants séparés ;
c. la production d'un signal de sortie électrique contenant des informations sur la grandeur et le temps de rétention pour les pics des constituants en utilisant ledit détecteur par spectrométrie de masse qui est connecté à la sortie de ladite colonne de CG ;
d. la comparaison de ladite sortie électrique avec le contenu de ladite mémoire en utilisant ledit microprocesseur ; et
e. l'indication de l'une des caractéristiques suivantes :
i. la présence dudit CSV dans ledit échantillon d'analyse, déterminée par comparaison avec les données présentes dans ladite mémoire du microprocesseur,
ii. la co-présence dans ledit échantillon d'analyse dudit CSV conjointement avec lesdites une ou plusieurs substances de marquage, ou
iii. l'absence de CSV et de substance de marquage dans l'échantillon d'analyse,
dans laquelle la co-présence dans l'échantillon d'analyse dudit CSV conjointement avec lesdites une ou plusieurs substances de marquage indique la présence de fissures ou d'autres défauts dans ladite barrière multicouche, tandis que la présence dudit CSV seul dans l'échantillon d'analyse indique la présence d'une nouvelle source de CSV.
